# EUROPEAN PATENT APPLICATION

(11) **EP 3 913 060 A1**
(43) Date of publication of application: **24.11.2021**
(21) Application number: 20305540.5
(22) Date of filing: 22.05.2020
(51) Int. Cl.: C12N 15/86

(54) **VECTORS ENCODING A GLUCOSE-6-PHOSPHATASE (G6PASE-A) FOR GENE THERAPY**

(71) Applicant: Genethon, 91000 Evry (FR); Institut National de la Santé et de la Recherche Médicale, 75013 Paris (FR); Universite d'Evry Val d'Essonne, 91000 Evry (FR); UNIVERSITE CLAUDE BERNARD - LYON 1, 69100 Villeurbanne (FR)
(72) Inventor: The designation of the inventor has not yet been filed
(74) Representative: Cabinet Beau de Loménie

(57) **Abstract**

The invention relates to a nucleic acid construct for the expression of a glucose-6-phosphatase-a (G6Pase-a) in a cell, the construct comprising a nucleic acid sequence encoding the G6Pase-a, wherein the nucleic acid sequence encoding the G6Pase-a is operably linked to a human alpha-1 antitrypsin (hAAT) promoter, a vector comprising the nucleic acid construct of the invention, a cell transformed with the nucleic acid construct or the vector of the invention, a composition comprising the nucleic acid construct, the vector or the cell of the invention, and the use thereof.

## Description

### Field of the invention

The invention relates to a nucleic acid construct for the expression of a glucose-6-phosphatase-a (G6Pase-a) in a cell, a vector comprising the nucleic acid construct useful in the treatment of glycogen storage disease Ia (GSD-Ia), wherein the nucleic acid sequence encoding the G6Pase-a is operably linked to a human alpha-1 antitrypsin (hAAT) promoter.

### Background of the invention

Glycogen storage disease type la (GSD-Ia or von Gierke disease) is caused by a deficiency in glucose-6-phosphatase-a (G6Pase-a), an enzyme that is expressed primarily in the liver, kidney, and intestine. G6Pase-a, encoded by the *G6PC* gene, is a hydrophobic protein anchored in the endoplasmic reticulum (ER) by nine transmembrane helices. This enzyme catalyzes the hydrolysis of glucose-6-phosphate (G6P) to glucose and inorganic phosphate in the terminal step of glycogenolysis and gluconeogenesis. Patients affected by GSD-Ia are unable to maintain glucose homeostasis and present with fasting hypoglycemia, growth retardation, hepatomegaly, nephromegaly, hyperlipidemia, hyperuricemia, and lactic academia.

Most of the time, hypoglycemia can be managed using dietary therapies that enable patients to attain near normal growth and pubertal development. However, the long-term clinical complications, and their underlying pathological processes, remain uncorrected. One of the most significant chronic risks is hepatocellular adenoma (HCA), that develops in 70-80% of GSD-I patients over 25 year-old. HCAs in GSD-Ia patients are multiple and non-encapsulated, with complications including local compression and intratumoral hemorrhage. In 10% of GSD-Ia patients, HCAs undergo malignant transformation to hepatocellular carcinoma (HCC).

Thus, a need exists for an improved therapy vector for the treatment of GSD-Ia and its associated complications.

Gene therapy studies using recombinant adeno-associated virus (AAV) carrying G6Pase-a have previously been performed in animal models of GSD-Ia. In particular, the prior art discloses the use of nucleic acid constructs comprising a nucleic acid sequence encoding the G6Pase-a which is operably linked to a *G6PC* promoter/enhancer (GPE).

Complications of treatment related to immune responses against the vector represent serious obstacles for the use of AAV vectors in gene therapy. The administration of lower doses of AAV vectors is, in general, associated to a lower activation of the immune response and a better stability of transgene expression [15]. In order to decrease the appearance of side effects associated with the use of AAV vectors, it is therefore preferable to administer the lowest amount of AAV vector that is sufficient to obtain a therapeutically effective effect in a subject.

Thus, there is a need for improved nucleic acid constructs that increases G6Pase-a expression and activity in gene therapy, allowing to decrease the amount of AAV vector that is necessary to achieve a therapeutic effect in a subject.

### Summary of the invention

The inventors have surprisingly shown that, in particular in an AAV gene therapy setting, a nucleic acid encoding G6Pase-a under the control of a hAAT promoter leads to increased G6Pase-a expression and G6Pase-a activity that permits phenotypical rescue in GSDIa mice compared to hGPE promoter. This was particularly unexpected in view of the prior art that used different forms of G6Pase native promoter (GPE) derived from different species to treat GSDIa.

In addition, the inventors have also shown that the same nucleic acid encoding G6Pase-a under the control of a hAAT promoter decreases the risk of tumor formation, such as HCA and HCC, when compared to hGPE promoter in gene therapy. This was also particularly unexpected in view of the prior art that predicts that promoters with higher activity are more likely to increase the risk of HCA and HCC after gene therapy [1].

Thus, in a first aspect, the invention relates to a nucleic acid construct for the expression of a glucose-6-phosphatase-a (G6Pase-a) in a cell, the construct comprising a nucleic acid sequence encoding the G6Pase-a, wherein the nucleic acid sequence encoding the G6Pase-a is operably linked to a human alpha-1 antitrypsin (hAAT) promoter.

In a second aspect, the invention relates to a vector comprising the nucleic acid construct of the invention.

In a third aspect, the invention relates to a cell transformed with the nucleic acid molecule of the invention or the vector of the invention.

In a fourth aspect, the invention relates to a composition comprising the nucleic acid molecule of the invention, the vector of the invention, or the cell of the invention.

In a fifth aspect, the invention relates to the nucleic acid construct, the vector, the cell or the composition of the invention, for use as a medicament, in particular for use in the treatment of glycogen storage disease Ia (GSD-Ia).

### Legends to the figures

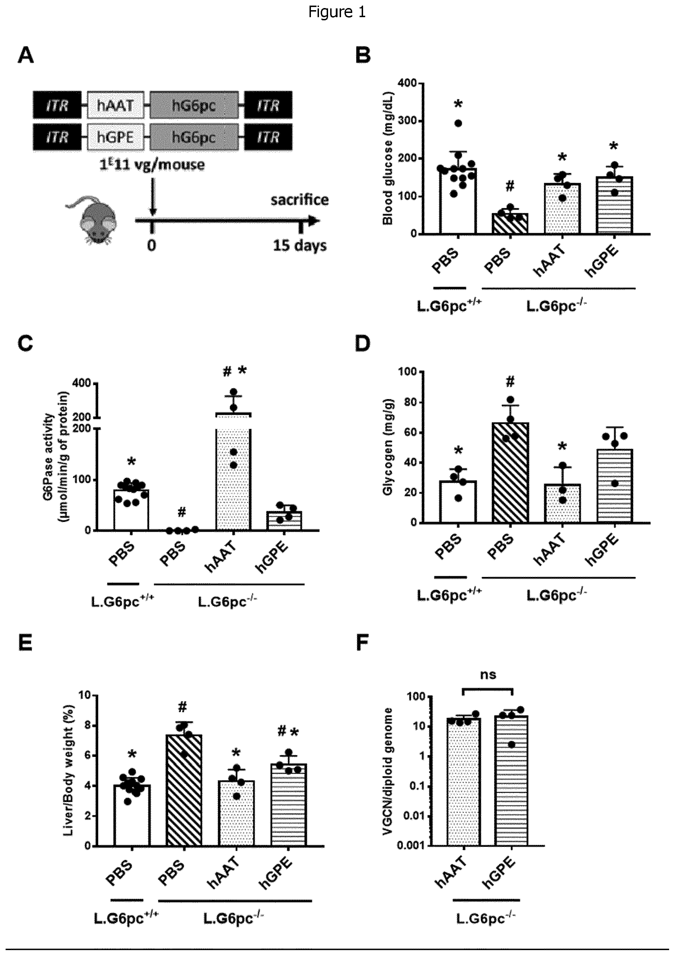

**Figure 1** shows the correction of the liver phenotype in GSD-Ia mice and WT mice after 15 days of treatment with AAV vectors or PBS. **A.** is the scheme of the protocol. **B.** shows the glycemia measured after 6h of fasting at the end of the protocol. **C.** shows G6Pase activity measured in liver tissues. **D.** shows glycogen content of liver tissues. **E.** shows hepatomegaly reported as the percentage of liver/body weight. **F.** shows the vector genome copy number per diploid genome measured in liver of AAV-treated mice. Statistical analyses were performed by ANOVA in A-E (# P<0.05 vs. PBS-injected L.G6pc^{+/+} mice; * P<0.05 vs. PBS-injected L.G6pc^{-/-} mice) and by t-test in F (ns, not significant).

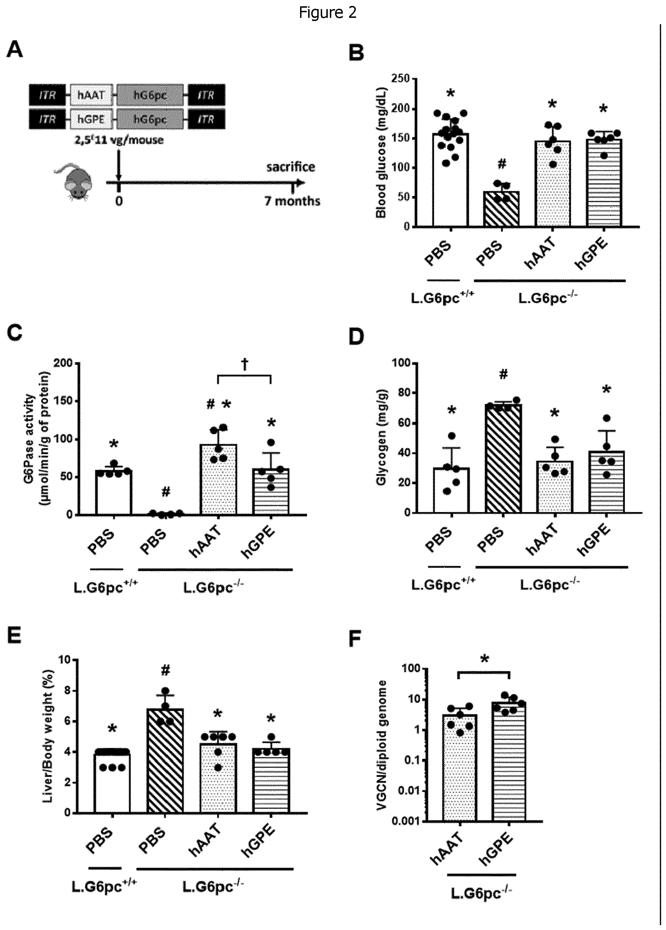

**Figure 2** shows the long-term correction of the liver phenotype in GSD-Ia mice. **A.** represents the scheme of the protocol. **B.** shows the glycemia measured after 6h of fasting at the end of the protocol, i.e. 7 months after vector injection. **C.** shows the G6Pase activity measured in liver tissues collected at sacrifice. **D.** shows the glycogen content measured on liver tissues. **E.** shows the hepatomegaly reported as the percentage of liver/body weight at sacrifice. **F.** shows the vector genome copy number per diploid genome measured in liver of AAV-treated mice. Statistical analyses were performed by ANOVA (# P<0.05 vs. PBS-injected L.G6pc^{+/+} mice; * P<0.05 vs. PBS-injected L.G6pc^{-/-} mice; † P<0.05 as indicated) and by t-test in F (* P<0.05).

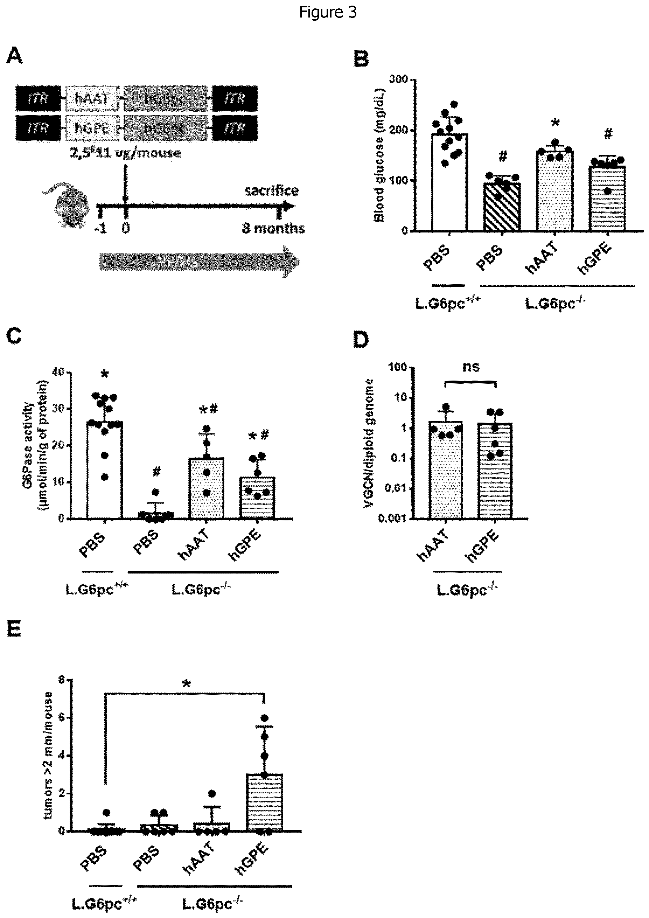

**Figure 3** shows that hGPE-directed gene therapy promotes hepatic tumor formation in L.G6pc^{-/-} mice fed a high fat high sucrose diet. **A.** represents the scheme of the protocol. **B.** shows the glycemia measured after 6h of fasting at the end of the protocol i.e. 8 months after vectors injection. **C.** shows the G6Pase-a activity assay performed on liver tissues collected at sacrifice. **D.** shows the vector genome copy number per diploid genome measured in liver. **E.** shows the number of tumors larger than 2 mm observed in PBS or AAV vector -treated L.G6pc^{-/-} mice 9 months after the start of the HF/HS regimen. Statistical analyses were performed by ANOVA in B and C (# P<0.05 vs. PBS-injected L.G6pc^{+/+} mice; * P<0.05 vs. PBS-injected L.G6pc^{-/-} mice), by t-test in D (ns, not significant) and by non-parametric ANOVA in E (* P<0.05 vs. PBS-injected L.G6pc^{+/+} mice).

### Detailed description of the invention

### Definitions

The terms "glucose-6-phosphatase alpha" or "G6Pase-a" relates to an enzyme encoded by the *G6pc* gene. This enzyme catalyzes the hydrolysis of glucose-6-phosphate (G6P) to glucose and inorganic phosphate in the terminal step of glycogenolysis and gluconeogenesis. According to the invention, G6Pase-a may be a wild-type G6Pase-a or a modified G6Pase-a, in particular a modified G6Pase-a having increased phosphohydrolase activity. Modified G6Pase-a are disclosed in WO2016106303 and [16]. For example, G6Pase-a may be SEQ ID NO: 1, SEQ ID NO: 12, or any the modified G6Pase-a having an amino acid sequence selected from SEQ ID NO: 29 to SEQ ID NO: 44.

According to the present invention, the "identity" is calculated by comparing two aligned sequences in a comparison window. The alignment of the sequences makes it possible to determine the number of positions (nucleotides or amino acids) in common for the two sequences in the comparison window. The number of positions in common is therefore divided by the total number of positions in the comparison window and multiplied by 100 to obtain the percentage of identity. The determination of the percentage of sequence identity can be carried out manually or by means of well-known computer programs. In a particular embodiment of the invention, the identity or the homology corresponds to at least one substitution, for example 1, 2, 3, 4, 5 substitutions, of an amino acid residue, without appreciable loss of interactive binding capacity. Preferably, the at least one substitution is a conservative amino acid substitution. By "conservative amino acid substitution", it is meant that an amino acid can be replaced with another amino acid having a similar side chain. Families of amino acid having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., glycine, cysteine, alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

According to the invention, the term "nucleic acid sequence" refers to a DNA or RNA molecule in single or double stranded form, particularly a DNA.

According to the invention, the term "nucleic acid sequence encoding a G6Pase-a" refers to a nucleic acid sequence encoding a G6Pase-a, either a wild-type G6Pase-a or a modified G6Pase-a. Modified nucleic acid sequence encoding a modified G6Pase-a are disclosed in reference [16] and WO2016106303. For example, a nucleic acid sequence encoding a wild-type G6Pase-a have the nucleotide sequence SEQ ID NO: 2 or SEQ ID NO: 3. For example, a nucleic acid sequence encoding a modified G6Pase-a have the nucleotide sequence SEQ ID NO: 4. For example, a nucleic acid sequence encoding a G6Pase-a may therefore be SEQ ID NO: 2, SEQ ID NO: 3, SEQ ID NO: 4, or any of the nucleic acid sequences encoding a modified G6Pase-a selected from SEQ ID NO: 13 to SEQ ID NO: 28.

The term "nucleic acid construct" refers to an artificially constructed segment of nucleic acid that is to be transplanted into a target cell for expressing a transgene, e.g. for the expression of a G6Pase-a in a cell. The nucleic acid construct may comprise one or more expression control nucleic acid sequences and/or other nucleic acid sequences improving the expression of G6Pase-a and/or nucleic acid sequences enhancing the secretion of G6Pase-a and/or nucleic acid sequences enhancing the tissue uptake of G6Pase-a, said nucleic acid sequences may be operably linked to the sequence encoding the transgene (e.g. G6Pase-a). As used herein, the term "operably linked" refers to a linkage of polynucleotide elements in a functional relationship. A nucleic acid sequence is "operably linked" when it is placed into a functional relationship with another nucleic acid sequence. For instance, a promoter, or another transcription regulatory nucleic acid sequence, is operably linked to a nucleic acid sequence encoding a transgene (e.g. G6Pase-a) if it affects the transcription of the nucleic acid sequence. Such expression control nucleic acid sequences are known in the art, such as promoters, enhancers (such as cis-regulatory modules (CRMs)), introns, polyA signals, etc.

The term "alpha-1 antitrypsin" or "AAT" relates to a protein belonging to the serpin superfamily. It is encoded in humans by the SERPINA1 gene. The term "hAAT" relates to the human AAT.

The term "promoter" refers to a region of DNA that directs/initiates transcription of a nucleic acid sequence (e.g. a gene). A promoter includes necessary nucleic acid sequences near the start site of transcription. Typically, promoters are located near the genes they transcribe. The term "hAAT promoter" relates to the promoter of hAAT, either a wild-type hAAT promoter or a modified hAAT promoter.

The term "vector" according to the invention means a vector suitable for a transgene expression in gene therapy, e.g. for G6Pase-a expression in gene therapy.

In the context of the present invention, the term "gene therapy" refers to treatment of a subject which involves delivery of a gene / nucleic acid into an individual's cells for the purpose of treating a disease.

The term "subject", "patient" or "individual", as used herein, refers to a human or non-human mammal (such as a rodent (mouse, rat), a feline, a canine, or a primate) affected or likely to be affected with a glycogen storage disease Ia (GSD-Ia). Preferably, the subject is a human, man or woman.

The term "glycogen storage disease" or "GSD" refers to a metabolic disorder caused by enzyme deficiencies affecting glycogen synthesis, glycogen breakdown or glycolysis, typically within muscles and/or liver cells. GSD is classified in different types, from GSD type 0 to GSD type XV. GSD-I consists of two autosomal recessive disorders, GSD-Ia and GSD-Ib. GSD-Ia results from a deficiency in glucose-6-phosphatase-a. Deficiencies in the glucose-6-phosphate transporter (G6PT) are responsible for GSD-Ib. According to the invention, the GSD is GSD-Ia (von Gierke's disease; OMIM # 232240).

The term "treating" or "treatment" means reversing, alleviating, inhibiting the progress of, or preventing the disorder or condition to which such term applies, or one or more symptoms of such disorder or condition. In particular, the treatment of the disorder may consist in treating dysfunctions in GSD, preferably improving the glycaemia control in a subject.

The term "pharmaceutically acceptable" means approved by a regulatory agency of the Federal or a state government or listed in the U.S. or European Pharmacopeia or other generally recognized pharmacopeia for use in animals and Humans.

A "pharmaceutical composition" means a composition comprising pharmaceutically acceptable carrier. For example, a carrier can be a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, sodium stearate, glycerol monostearate, talc, sodium chloride, dried skim milk, glycerol, propylene glycol, water, ethanol and the like. When the pharmaceutical composition is adapted for oral administration, the tablets or capsules can be prepared by conventional means with pharmaceutically acceptable excipients such as binding agents (e.g. pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p- hydroxybenzoates or sorbic acid). The preparations may also contain buffer salts, flavoring, coloring and sweetening agents as appropriate. The composition according to the invention is preferably a pharmaceutical composition.

### Nucleic acid construct

The invention relates to a nucleic acid construct for the expression of a glucose-6-phosphatase (G6Pase-a) in a cell, the construct comprising a nucleic acid sequence encoding the G6Pase-a, wherein the nucleic acid sequence encoding the G6Pase-a is operably linked to a human alpha-1 antitrypsin (hAAT) promoter.

According to the invention, the nucleic acid sequence may encode a wild-type G6Pase-a, e.g. the G6Pase-a having the amino acid sequence SEQ ID NO: 1, or a modified G6Pase-a, preferably a modified G6Pase-a having increased phosphohydrolase activity, e.g. a G6Pase-a comprising or having an amino acid sequence at least 90% identical to SEQ ID NO: 1. The modified G6Pase-a can include one or more amino acid modification(s), such as substitution or deletion, so long as the protein retains enzymatic activity. In some embodiments, the modified G6Pase-a comprises a serine to cysteine substitution at amino acid 298 of human G6Pase-a (the amino acid sequence of wild type human G6Pase-a is set forth herein as SEQ ID NO: 1). The modified G6Pase-a can include modifications at other residues so long as the protein retains enzymatic activity. For example, the modified G6Pase-a can include substitutions at other residues, such residues include positions 3, 54, 139, 196, 199, 242, 247, 292, 301, 318, 324, 332, 347, 349, 350 and/or 353 of the human G6Pase-a (set forth as SEQ ID NO: 1). Modified G6Pase-a are disclosed in WO2016/106303 and [16]. For example, G6Pase-a may be SEQ ID NO: 1, SEQ ID NO: 12, or any modified G6Pase-a having an amino acid sequence selected from SEQ ID NO: 13 to SEQ ID NO: 28.

In some embodiments, the G6Pase-a comprises or has an amino acid sequence at least 90%, at least 91%, at least 92%, at least 93%, at least 94%, at least 95%, at least 96%, at least 97%, at least 98%, at least 99%, or 100% identical to SEQ ID NO: 1.

The nucleic acid sequence encoding the G6Pase-a may therefore encode either a wild-type G6Pase-a or a modified G6Pase-a. Nucleic acid sequence encoding a modified G6Pase-a may be any of the nucleic acid sequences selected from SEQ ID NO: 29 to SEQ ID NO: 44.

In some embodiments, the nucleic acid sequence encoding the G6Pase-a comprises a nucleotide sequence having at least 90%, at least 91%, at least 92%, at least 93%, at least 94%, at least 95%, at least 96%, at least 97%, at least 98%, at least 99%, or 100% identity with SEQ ID NO: 2.

For example, a nucleic acid sequence encoding a G6Pase-a may therefore be SEQ ID NO: 2, SEQ ID NO: 3, SEQ ID NO: 4, or any of nucleic acid sequences n°6-7 of WO2016106303.

The nucleic acid sequence encoding a G6Pase-a is operably linked to a human alpha-1 antitrypsin (hAAT) promoter.

According to the invention, the hAAT promoter may be a wild-type hAAT promoter, e.g. the hAAT promoter having the nucleic acid sequence SEQ ID NO: 8, or a modified hAAT promoter, e.g. a hAAT promoter comprising or having an amino acid sequence at least 90% identical to SEQ ID NO: 8. The modified hAAT promoter can include one or more amino acid modification(s), such as substitution or deletion, so long as the promoter still directs/initiates transcription of the G6Pase-a.

In some embodiments, the hAAT promoter comprises or has a nucleotide sequence having at least 90%, at least 91%, at least 92%, at least 93%, at least 94%, at least 95%, at least 96%, at least 97%, at least 98%, at least 99%, or 100% identity with SEQ ID NO: 8.

In a particular embodiment, the nucleic acid construct comprises an intron, in particular an intron placed between the hAAT promoter and the nucleic acid sequence encoding a G6Pase-a. An intron may be introduced to increase mRNA stability and the production of G6Pase-a.

In a particular embodiment, the nucleic acid construct of the invention comprises, in the 5' to 3' orientation, the hAAT promoter optionally preceded by an enhancer, such as ApoE enhancer (e.g. SEQ ID NO: 9), the nucleic acid sequence encoding the G6Pase-a and a polyadenylation signal (such as the bovine growth hormone polyadenylation signal, the HBB2 polyadenylation signal, the SV40 polyadenylation signal, or another naturally occurring or artificial polyadenylation signal). In a particular embodiment, the nucleic acid construct of the invention comprises, in the 5' to 3' orientation, the hAAT promoter optionally preceded by an enhancer, such as ApoE enhancer (e.g. SEQ ID NO: 9), an intron (in particular an intron as defined above), a nucleic acid molecule encoding the G6Pase-a, and a polyadenylation signal.

In a particular embodiment, the nucleic acid construct comprises or has a nucleotide sequence having at least 90%, at least 91%, at least 92%, at least 93%, at least 94%, at least 95%, at least 96%, at least 97%, at least 98%, at least 99%, or 100% identity with SEQ ID NO: 7 or SEQ ID NO: 11.

The term nucleic acid construct is to be transferred into a target cell for the expression of a G6Pase-a in said cell. Preferably, said target cell is a liver cell, a kidney cell or an intestinal cell.

### Vector

The invention also relates to a vector comprising the nucleic acid construct of the invention.

In some embodiments, the vector is a plasmid vector. In another embodiment, the vector is a nanoparticle containing the nucleic acid construct of the invention. In another embodiment, the vector is a system based on transposons, allowing integration of the nucleic acid construct of the invention in the genome of the target cell, such as the hyperactive Sleeping Beauty (SB100X) transposon system [2]. In another embodiment, the vector is a viral vector suitable for gene therapy. The vector may target any cell of interest such as liver cells, kidney cells or intestinal cells.

In a preferred embodiment, the vector is a viral vector, such as lentiviral vector or adeno-associated virus (AAV) vector, preferably an AAV vector. In a particularly preferred embodiment, the AAV vector implemented in the practice of the present invention is AAV8 or AAV9, preferably AAV8.

Thus, the nucleic acid construct of the invention may also contain sequences suitable for producing an efficient viral vector, as it is well disclosed in the art. In a preferred embodiment, the nucleic acid construct is inserted in a retroviral vector, such as a lentiviral vector, or an AAV vector, such as a single-stranded or double-stranded self-complementary AAV vector. In a much preferred embodiment of the present invention, the viral vector is an AAV vector, such as an AAV vector suitable for transducing liver cells, more particularly an AAV-1, AAV-2 and AAV-2 variants (such as the quadruple-mutant capsid optimized AAV-2 comprising an engineered capsid with Y44+500+730F+T491V changes, disclosed in [3]), AAV-3 and AAV-3 variants (such as the AAV3-ST variant comprising an engineered AAV-3 capsid with two amino acid changes, S663V+T492V, disclosed in Vercauteren et al. [4], AAV-3B and AAV-3B variants, AAV-4, AAV-5, AAV-6 and AAV-6 variants (such as the AAV-6 variant comprising the triply mutated AAV-6 capsid Y731F/Y705F/T492V form disclosed in [5], AAV-7, AAV-8, AAV-9, AAV-10 such as AAV-cy10 and AAV-rh10, AAV-rh74, AAV-dj, Anc80, LK03, AAV-2i8, porcine AAV serotypes such as AAV-po4 and AAV-po6, etc., vector or a retroviral vector such as a lentiviral vector and an alpha-retrovirus. As it is known in the art, depending on the specific viral vector considered for use, additional suitable sequences will be introduced in the nucleic acid construct of the invention for obtaining a functional viral vector. Suitable sequences include AAV ITRs for an AAV vector, or LTRs for lentiviral vectors. As such, the invention also relates to a nucleic acid construct as described above, flanked by an ITR or an LTR on each side.

In addition, other non-natural engineered variants and chimeric AAV can also be useful. AAV viruses may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus. Desirable AAV fragments for assembly into vectors include the cap proteins, including the vp1, vp2, vp3 and hypervariable regions, the rep proteins, including rep 78, rep 68, rep 52, and rep 40, and the sequences encoding these proteins. These fragments may be readily utilized in a variety of vector systems and host cells. AAV-based recombinant vectors lacking the Rep protein integrate with low efficacy into the host's genome and are mainly present as stable circular episomes that can persist for years in the target cells. Alternatively to using AAV natural serotypes, artificial AAV serotypes may be used in the context of the present invention, including, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid.

In the context of the present invention, the AAV vector comprises an AAV capsid able to transduce the target cells of interest, in particular hepatocytes. In a further particular embodiment, the AAV vector is a pseudotyped vector, i.e. its genome and capsid are derived from AAVs of different serotypes. For example, the pseudotyped AAV vector may be a vector whose genome is derived from one of the above mentioned AAV serotypes, and whose capsid is derived from another serotype.

According to a particular embodiment, the AAV capsid is selected from AAV-1, -2, AAV-2 variants (such as the quadruple-mutant capsid optimized AAV-2 comprising an engineered capsid with Y44+500+730F+T491V changes, disclosed in Ling et al., 2016, -3 and AAV-3 variants (such as the AAV3-ST variant comprising an engineered AAV3 capsid with two amino acid changes, S663V+T492V, disclosed in Vercauteren et al., 2016, -3B and AAV-3B variants, -4, -5, -6 and AAV-6 variants (such as the AAV6 variant comprising the triply mutated AAV6 capsid Y731F/Y705F/T492V form disclosed in Rosario et al., 2016), -7, -8, -9 and AAV-9 variants (such as AAVhu68), - 2G9, -10 such as -cy10 and -rh10, -rh39, -rh43, -rh74, -dj, Anc80, LK03, AAV.PHP, AAV2i8, porcine AAV such as AAVpo4 and AAVpo6, and tyrosine, lysine and serine capsid mutants of AAV serotypes. In addition, the AAV capsid is selected from other non-natural engineered variants (such as AAV-spark100), chimeric AAV or AAV serotypes obtained by shuffling, rationale design, error prone PCR, and machine learning technologies. In a particular embodiment, the Cap gene encodes VP capsid proteins derived from at least two different AAV serotypes, or encodes at least one chimeric VP protein combining VP protein regions or domains derived from at least two AAV serotypes. For example a chimeric AAV capsid can derive from the combination of an AAV8 capsid sequence with a sequence of an AAV serotype different from the AAV8 serotype, such as any of those specifically mentioned above. In another embodiment, the capsid of the AAV vector comprises one or more variant VP capsid proteins such as those described in WO2015013313, in particular the RHM4-1, RHM15-1, RHM15-2, RHM15-3/RHM15-5, RHM15-4 and RHM15-6 capsid variants. In a particular embodiment, the capsid of the AAV vector is a hybrid between AAV serotype 9 (AAV9) and AAV serotype 74 (AAVrh74) capsid proteins. For example, the AAV serotype may be a -rh74-9 serotype as disclosed in WO2019/193119 (such as the Hybrid Cap rh74-9 serotype described in examples of WO2019/193119; a rh74-9 serotype being also referred to herein as "-rh74-9", "AAVrh74-9" or "AAV-rh74-9") or a -9-rh74 serotype as disclosed in WO2019/193119 (such as the Hybrid Cap 9-rh74 serotype described in the examples of WO2019/193119; a -9-rh74 serotype being also referred to herein as "-9-rh74", "AAV9-rh74", "AAV-9-rh74", or "rh74-AAV9"). For example, the capsid of the AAV vector is a peptide-modified hybrid between AAV serotype 9 (AAV9) and AAV serotype 74 (AAVrh74) capsid proteins, as described in PCT/EP2019/076958, such as an AAV9-rh74 hybrid capsid or AAVrh74-9 hybrid capsid modified with the P1 peptide described in the examples of PCT/EP2019/076958For another example, the AAV serotype may be a hybrids AAV2/13 as disclosed in PCT/EP2020/061380.

For example, the genome of the pseudotyped vector may have a capsid derived from the AAV8, AAV9, AAVrh74 or AAV2i8 serotype, and its genome may be derived from and different serotype. In a particular embodiment, the AAV vector has a capsid of the AAV8, AAV9 or AAVrh74 serotype, in particular of the AAV8 or AAV9 serotype, more particularly of the AAV8 serotype.

In a specific embodiment, wherein the vector is for use in delivering the transgene to muscle cells, the AAV vector may be selected, among others, in the group consisting of AAV8, AAV9 and AAVrh74.

In another specific embodiment, wherein the vector is for use in delivering the transgene to liver cells, the AAV vector may be selected, among others, in the group consisting of AAV5, AAV8, AAV9, AAV-LK03, AAV-Anc80 and AAV3B.

In another embodiment, the capsid is a modified capsid. In the context of the present invention, a "modified capsid" may be a chimeric capsid or capsid comprising one or more variant VP capsid proteins derived from one or more wild-type AAV VP capsid proteins.

In a particular embodiment, the AAV vector is a chimeric vector, i.e. its capsid comprises VP capsid proteins derived from at least two different AAV serotypes, or comprises at least one chimeric VP protein combining VP protein regions or domains derived from at least two AAV serotypes. Examples of such chimeric AAV vectors useful to transduce liver cells are described in [6] and in [7]. For example a chimeric AAV vector can derive from the combination of an AAV8 or AAV9 capsid sequence with a sequence of an AAV serotype different from the AAV8 or AAV9 serotype, such as any of those specifically mentioned above. In another embodiment, the capsid of the AAV vector comprises one or more variant VP capsid proteins such as those described in WO2015013313, in particular the RHM4-1, RHM15-1, RHM15-2, RHM15-3/RHM15-5, RHM15-4 and RHM15-6 capsid variants, which present a high liver tropism.

In another embodiment, the modified capsid can be derived also from capsid modifications inserted by error prone PCR and/or peptide insertion (e.g. as described in [8], or in [9]. In addition, capsid variants may include single amino acid changes such as tyrosine mutants (e.g. as described in [10]). Another example is the fusion of Anthopleurin-B to the N-terminus of AAV VP2 capsid protein described in [11].

In addition, the genome of the AAV vector may either be a single stranded or self-complementary double-stranded genome [12]. Self-complementary double-stranded AAV vectors are generated by deleting the terminal resolution site (trs) from one of the AAV terminal repeats. These modified vectors, whose replicating genome is half the length of the wild type AAV genome, have the tendency to package DNA dimers.

In a preferred embodiment, the AAV vector implemented in the practice of the present invention has a single stranded genome, and further preferably comprises an AAV8, AAV9, AAVrh74 or AAV2i8 capsid, in particular an AAV8, AAV9 or AAVrh74 capsid, such as an AAV8 or AAV9 capsid, more particularly an AAV8 capsid.

### Cell

The invention also relates to a cell transformed with the nucleic acid molecule of any one of claims 1 to 5 or the vector of any one of claims 6 to 9.

For example, the host cell can be a cell (or cell line) appropriate for production of the vector, e.g. for production of AAV. In some examples, the host cell is a mammalian cell, such as a HEK-293, BHK, Vero, RD, HT-1080, A549, Cos-7, ARPE-19, or MRC-5 cell.

The host cell can also be a cell which is the target for a gene therapy, such as a liver cell, a kidney cell or an intestinal cell.

In some embodiments, the cell is an isolated cell.

### Composition

The invention also relates to a composition comprising the nucleic acid construct of the invention, the vector of the invention, or the cell of the invention. The composition of the invention is preferably a pharmaceutical composition.

The composition can take the form of solutions, suspensions, emulsions, tablets, pills, capsules, powders, sustained-release formulations and the like.

Such composition will contain a therapeutically effective amount of the nucleic acid construct of the invention, of the vector of the invention, or of the cell of the invention, preferably in purified form, together with a suitable amount of carrier so as to provide the form for proper administration to the subject. In a particular embodiment, the nucleic acid construct, vector or cell of the invention is formulated in a composition comprising phosphate-buffered saline and supplemented with 0.25% human serum albumin. In another particular embodiment, the nucleic acid construct, vector or cell of the invention is formulated in a composition comprising ringer lactate and a non-ionic surfactant, such as pluronic F68 at a final concentration of 0.01-0.0001%, such as at a concentration of 0.001%, by weight of the total composition. The formulation may further comprise serum albumin, in particular human serum albumin, such as human serum albumin at 0.25%. Other appropriate formulations for either storage or administration are known in the art, in particular from WO 2005/118792.

In a preferred embodiment, the composition is formulated in accordance with routine procedures as a pharmaceutical composition adapted for intravenous or intrathecal administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection.

### Method of treatment

The invention also relates to the nucleic acid construct of the invention, the vector of the invention, the cell of the invention or the composition of the invention, for use as a medicament.

The invention also relates to the nucleic acid construct of the invention, the vector of the invention, the cell of the invention or the composition of the invention, for use in the treatment of glycogen storage disease Ia (GSD-Ia).

The nucleic acid construct, the vector, the cell or the composition of the invention may be administered to a subject by any effective route. Exemplary routes of administration include, but are not limited to, injection (such as subcutaneous, intramuscular, intradermal, intraperitoneal, intrathecal, and intravenous), oral, intraductal, sublingual, rectal, transdermal, intranasal, vaginal and inhalation routes.

The nucleic acid construct, the vector, the cell or the composition of the invention may be administered to a subject in a therapeutically effective amount, i.e. in an amount sufficient to achieve the desired effect in a subject, or in the cell, being treated. The effective amount of the nucleic acid construct, the vector, the cell or the composition of the invention will be dependent on several factors, including, but not limited to the subject or cells being treated, and the manner of administration.

The amount of the therapeutic (i.e. a nucleic acid construct, vector or cell) of the invention which will be effective in the treatment of a disease, such as GSD-Ia, can be determined by standard clinical techniques. In addition, *in vivo* and/or *in vitro* assays may optionally be employed to help predict optimal dosage ranges. The precise dose to be employed in the composition will also depend on the route of administration, and the seriousness of the disease, and should be decided according to the judgment of the practitioner and each patient's circumstances. The dosage of the nucleic acid, the vector or the cell administered to the subject in need thereof will vary based on several factors including, without limitation, the route of administration, the specific disease treated, the subject's age or the level of expression necessary to require the therapeutic effect. One skilled in the art can readily determine, based on its knowledge in this field, the dosage range required based on these factors and others. In case of a treatment comprising administering a viral vector, such as an AAV vector, to the subject, typical doses of the vector are of at least 1x10⁸ vector genomes per kilogram body weight (vg/kg), such as at least 1x10⁹ vg/kg, at least 1x10¹⁰ vg/kg, at least 1x10¹¹ vg/kg, at least 1x10¹² vg/kg, at least 1x10¹³ vg/kg, or at least 1x10¹⁴ vg/kg.

The therapeutic (i.e. a nucleic acid construct, vector or cell) of the invention can be administered in a single dose, or in multiple doses (such as 2, 3, 4, 5, 6, 7, 8, 9 or 10 doses) as needed for the desired therapeutic results.

### Examples

### Materials and methods

We have prepared two nucleic acid constructs (hereafter "transgene expression cassettes") comprising the human G6pc gene encoding G6Pase-a, under the control of different promoters:
- The alpha-1-anti-trypsin (hAAT) promoter. The nucleic acid sequence of the expression cassette is SEQ ID NO: 11.
- The endogenous promoter of the human G6pc gene (hGPE). The nucleic acid sequence of the expression cassette is SEQ ID NO: 10.

The transgene expression cassettes are represented in Figure 1A.

The two transgene expression cassettes were pseudotyped in AAV9 to obtain two different AAV vectors, namely AAV9-hAAT-hG6PC and AAV9-hGPE-hG6PC.

The two vectors or PBS (negative control) were independently tested in a liver-specific G6pc knockout mouse model (L.G6pc-/-, [13]), i.e. GSD-Ia mice. As a positive control, WT mice (L.G6pc+/+) were infused with PBS.

### In vivo studies

Mice were fed either a standard chow diet (A04 diet, Safe) or a high fat/high sucrose diet (made by INRAE, Jouy en Josas), known to accelerate hepatic tumor development in L.G6pc-/- mice. Proneness to tumor formation was induced in mice by feeding them with a modified chow diet composed of 36% fat (INRA) [14].

AAV vectors were administered intravenously via the tail vein of L.G6pc-/- mice. Wild type mice (C57Bl/6J mice, Charles Rivers) were injected with PBS via the tail vein. Glycemia was measured on peripheral blood after a 6-hour fasting using a glucometer (Roche Diagnostic). At sacrifice, livers were harvested and weighed to measure the liver/body weight ratio and snap-frozen for further evaluations.

Tumors were evaluated in GSDIa mice at sacrifice by visual inspection. Only tumors larger than 2 mm were considered in the count.

### AAV production

HEK293T cells were grown in suspension in 250 mL of serum-free medium. The cells were transfected with 3 plasmids: i) a transgene plasmid, containing AAV2 ITRs flanking an expression cassette ii) the helper plasmid pXX6, containing adenoviral sequences necessary for AAV production, and iii) a plasmid containing AAV Rep and Cap genes, defining the serotype of AAV. Two days after transfection, the cells were lysed to release the AAV particles.

The viral lysate was purified by affinity chromatography. Viral genomes were quantified by a TaqMan real-time PCR assay using primers and probes corresponding to the ITRs of the AAV vector genome [17].

### G6Pase enzyme activity measurement

G6Pase enzyme activity was measured in homogenates from freeze-clamped livers as already reported in [13]. Briefly, tissues homogenates were incubated with glucose-6-phosphate (Sigma) for fifteen minutes at 37°C. The reaction was stopped by adding trichloroacetic acid and the released phosphate was measured by complexation with ammonium molybdate and citrate arsenite. The resulting absorbance was measured on at 700 nm.

### Measurement of glycogen content

Glycogen content was measured indirectly in tissue homogenates as the glucose released after total digestion with Aspergillus Niger amyloglucosidase (Sigma). Samples were incubated for 20 min at 95°C in the presence of 0.3 M NaOH and then cooled at 4°C. Samples were then added with amyloglucosidase and incubated at 37°C for 90 minutes. The glucose released was determined with a commercial glucose assay kit.

### Vector genome copy number (VGCN) quantification

For vector genome copy number (VGCN) quantification in samples, DNA was extracted from samples using KingFisher (Thermo Fisher Scientific). Real-time PCR was performed on 1µL of DNA, using the protocol for AAV vectors titration described above. Exon Mex5 of titin gene was used as genomic DNA loading control.

### Example 1 : Correction of the liver phenotype in GSD-Ia mice 15 days after intravenous injection of AAV vectors expressing human G6Pase-a

The two AAV9 vectors were independently injected in L.G6pc^{-/-} mice fed a standard diet at the dose of 1x10¹¹ vg/mouse (Figure 1A).

Fifteen days after vectors injection, the concentration of glucose in the blood was measured after 6 hours of fasting. Glycaemia was completely normalized in mice injected with the two vectors (Figure 1B).

G6PC activity was then measured in liver. The AAV9-hAAT-hG6PC vector achieved supraphysiological activity and showed the highest activity when compared to AAV9-hGPE-hG6PC vector (Figure 1C).

Glycogen concentration was also measured. Complete correction of glycogen accumulation and hepatomegaly was obtained with the AAV9-hAAT-hG6PC but not with the vector bearing the hGPE promoter (Figure 1D, E respectively), reflecting the higher G6Pase activity achieved in mice treated with the AAV9 vector bearing hAAT promoter. The similar vector genome copy numbers per diploid genome measured in mice injected with the two AAV vectors (Figure 1F) indicates a similar transduction efficacy in liver.

### Example 2: long-term correction of the liver phenotype in GSD-Ia mice after intravenous injection of AAV vectors expressing human G6Pase-a

To assess the long-term efficacy of the two vectors, we performed a 7-months study. Vectors were injected in L.G6pc^{-/-} mice fed a standard diet at a dose of 2.5x10¹¹ vg/mouse (Figure 2A). Seven months after vectors injection, the concentration of glucose in the blood was measured after 6 hours of fasting. Glycemia was corrected in all the animals who received the G6PC-expressing vectors (Figure 2B). Importantly, L.G6pc^{-/-} mice treated with AAV9-hAAT-hG6PC showed supraphysiological liver G6Pase-a activity that was significantly higher of the activity measured in AAV9-hGPE-hG6PC -treated L.G6pc^{-/-} mice (Figure 2C).

Glycogen accumulation and hepatomegaly were completely rescued in animals injected with the two vectors (Figure 2D, E). Higher vector genome copy numbers per diploid genome were measured in mice injected with the AAV9-hGPE-hG6PC vector (Figure 2F) possibly reflecting a slightly lower liver transduction achieved with the AAV9-hAAT-hG6PC vector.

Taken together, these results indicate that G6Pase-a expression is increased with the hAAT promoter. Thus, hAAT promoter is suitable for AAV gene therapy for GSD-Ia with a potency superior to the hGPE promoter.

### Example 3: hGPE-directed gene therapy promotes hepatic tumor formation in L.G6DC-/- mice fed a high fat/hiah sucrose diet.

Adenoma formation is one of the hallmarks of GSD-Ia in humans, reported in most of the affected individuals in the second and third decades of life. In L.G6pc^{-/-} mice, almost all the mice fed a standard diet developed liver adenomas by 18 months of age [13]. This slow process can be accelerated by high fat/high sucrose (HF/HS) diet. Approximately 85 % of L.G6pc^{-/-} mice fed a HF/HS diet developed multiple hepatic tumors at nine months of age [14]. Thus, L.G6pc^{-/-} mice fed with HF/HS diet represent a robust model to evaluate the efficacy and the safety of gene replacement strategies in the prevention of tumors formation in GSDIa.

We therefore tested the AAV9-hAAT-hG6PC and the AAV9-hGPE-hG6PC vectors in L.G6pc-/-mice fed a HF/HS diet (Figure 3A). Eight months after treatment, only the vector bearing the hAAT promoter completely rescued glycemia (Figure 3B). Under HF/HS diet, the levels of G6Pase activity in AAV-treated animals were significantly lower than those measured in L.G6pc^{+/+} animals. Although AAV9-hAAT-hG6PC -treated animals showed higher G6Pase activity levels, statistical significance was not reached when compared to L.G6pc^{-/-} mice treated with AAV9-hGPE-hG6PC vector (Figure 3C). Similar vector genome copies per diploid genome were measured in AAV-treated mice (Figure 3D).

Interestingly, necropsy of the mice revealed a higher number of tumors in the livers of mice treated with the hGPE-bearing AAV vectors compared to those that received the AAV9-hAAT-hG6PC (Figure 3E). These results suggest that the use of AAV9-hAAT-hG6PC may decrease the transformation rate of hepatocytes thus resulting in a decreased frequency of adenomas compared to AAV9-hGPE-hG6PC.

### References cited under the form "[reference number]"

[1] Chandler et al., Vector design influences hepatic genotoxicity after, The Journal of Clinical Investigation, 2015
[2] Mates et al. Nat Genet. 2009 Jun;41(6):753-61. doi: 10.1038/ng.343
[3] Ling et al., 2016 Jul 18, Hum Gene Ther Methods.
[4] Vercauteren et al., 2016, Mol. Ther. Vol. 24(6), p. 1042
[5] Rosario et al., 2016, Mol Ther Methods Clin Dev. 3, p.16026
[6] Shen et al., 2007 Molecular Therapy, volume 15, issue 11, pages 1955-1962
[7] Tenney et al., Virology, volumes 454-455, April 2014, pages 227-236
[8] Bartel et al., Front. Microbiol., 04 October 2011 https://doi.org/10.3389/fmicb.2011.00204
[9] Michelfelder et al. (PLoS ONE, 2009, 4, e5122
[10] Zhong et al., PNAS June 3, 2008 105 (22) 7827-7832; https://doi.org/10.1073/pnas.0802866105
[11] Finet et al., Virology, 2018, 513, 43-51.
[12] McCarty et al., 2003 Gene Therapy Dec;10(26):2112-8.
[13] Mutel et al., Targeted deletion of liver glucose-6 phosphatase mimics glycogen storage disease type 1a including development of multiple adenomas, Journal of Hepatology, 2011 Mar;54(3):529-37. doi: 10.1016/j.jhep.2010.08.014. Epub 2010 Oct 1.
[14] Gjorgjieva M. et al, Dietary exacerbation of metabolic stress leads to accelerated hepatic carcinogenesis in glycogen storage disease type Ia, J. Hepatol. 2018; Nov;69(5): 1074-1087. doi: 10.1016/j.jhep.2018.07.017. Epub 2018 Sep 5.
[15] George L. et al, Hemophilia B Gene Therapy with a High-Specific-Activity Factor IX Variant. N Engl J Med. 2017 Dec 7;377(23):2215-2227. doi: 10.1056/NEJMoa1708538.
[16] Zhang L. et al, An evolutionary approach to optimizing glucose-6-phosphatase-α enzymatic activity for gene therapy of glycogen storage disease type Ia. J Inherit Metab Dis. 2019 May;42(3):470-479. doi: 10.1002/jimd.12069. Epub 2019 Feb 22
[17] Rohr et al., J. Virol. Methods, 2002, 106, 81-88)

### Sequence listing

| **SEQ ID NO :** | **Description** | **Sequence** |
|---|---|---|
| **1** | hG6PCwt (amino acid) | |
| **2** | hG6PCwt (nucleic acid) (Encoding SEQ ID NO : 1) | atggaggaaggaatgaatgttctccatgactttgggatccagtcaacacattacctccaggt gaattaccaagactcccaggactggttcatcttggtgtccgtgatcgcagacctcaggaatg ccttctacgtcctcttccccatctggttccatcttcaggaagctgtgggcattaaactcctttgg gtagctgtgattggagactggctcaacctcgtctttaagtggattctctttggacagcgtcca tactggtgggttttggatactgactactacagcaacacttccgtgcccctgataaagcagttc cctgtaacctgtgagactggaccagggagcccctctggccatgccatgggcacagcaggt gtatactacgtgatggtcacatctactctttccatctttcagggaaagataaagccgacctac agatttcggtgcttgaatgtcattttgtggttgggattctgggctgtgcagctgaatgtctgtc tgtcacgaatctaccttgctgctcattttcctcatcaagttgttgctggagtcctgtcaggcatt gctgttgcagaaactttcagccacatccacagcatctataatgccagcctcaagaaatatttt ctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **3** | hG6PCco (nucleic acid) (Codon optimized - encoding SEQ ID NO : 1) | atggaggagggaatgaatgtgctgcacgacttcgggattcagtctacccactatctccaag tcaattatcaagacagtcaagattggttcatcctcgtctccgttatagctgacctcaggaatg ctttttatgtactgttcccaatatggtttcatctgcaagaagcagttggaattaaactgctgtg ggtggccgtaatcggagactggttgaacctggtgttcaaatggatcctttttggtcagaggc catattggtgggtcttggacaccgactattatagcaacacatctgtacccctgataaagcaa ttccctgtaacgtgtgaaactgggcctgggtcacccagtggacacgcgatggggactgcc ggggtttactacgtaatggttactagcacgcttagcattttccagggcaagatcaaaccgac ttatcgcttccgatgcctcaatgtgatcctctggctgggattctgggcggttcaactgaacgt |
| | | atgcctctcacggatttatctggctgcacatttcccgcatcaagtagtggctggtgtgttgtct ggcatagcagtggctgaaacattctcacacattcattctatttacaacgcttcattgaaaaaa tactttctgattactttttttcttttcagctttgcaattggcttttacttgctgcttaaaggtcttggc gtcgacctgctctggactcttgagaaagcgcaacgctggtgcgaacaacccgagtgggtc cacatagatacgaccccgttcgcgtctcttctgaaaaatctcggtaccctcttcggactggga cttgctttgaattcttcaatgtatcgggaatcctgcaagggcaaactgtccaagtggttgccc tttcgcctttcaagcattgtcgcttcccttgtgctcctgcacgtcttcgactcactgaagccgcc gagccaagtcgagctcgtgttttacgtgttgtctttctgtaaaagtgccgtcgtgccactcgc cagtgtgagcgtgataccttattgcctcgcacaggtgcttgggcagcctcataagaagtctc tgtaa |
| **4** | hG6PCmut (nucleic acid) (identical to SEQ ID NO: 2 except for a c >g mutation at position 893) (Encoding SEQ ID NO : 12) | atggaggaaggaatgaatgttctccatgactttgggatccagtcaacacattacctccaggt gaattaccaagactcccaggactggttcatcttggtgtccgtgatcgcagacctcaggaatg ccttctacgtcctcttccccatctggttccatcttcaggaagctgtgggcattaaactcctttgg gtagctgtgattggagactggctcaacctcgtctttaagtggattctctttggacagcgtcca tactggtgggttttggatactgactactacagcaacacttccgtgcccctgataaagcagttc cctgtaacctgtgagactggaccagggagcccctctggccatgccatgggcacagcaggt gtatactacgtgatggtcacatctactctttccatctttcagggaaagataaagccgacctac agatttcggtgcttgaatgtcattttgtggttgggattctgggctgtgcagctgaatgtctgtc tgtcacgaatctaccttgctgctcattttcctcatcaagttgttgctggagtcctgtcaggcatt gctgttgcagaaactttcagccacatccacagcatctataatgccagcctcaagaaatatttt ctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctgtattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **5** | hG6PCmutco (Nucleic acid) (Codon optimized - encoding SEQ ID NO : 12) | atggaggaaggaatgaatgttctccatgactttgggatccagtcaacacattacctccaggt gaattaccaagactcccaggactggttcatcttggtgtccgtgatcgcagacctcaggaatg ccttctacgtcctcttccccatctggttccatcttcaggaagctgtgggcattaaactcctttgg gtagctgtgattggagactggctcaacctcgtctttaagtggattctctttggacagcgtcca tactggtgggttttggatactgactactacagcaacacttccgtgcccctgataaagcagttc cctgtaacctgtgagactggaccagggagcccctctggccatgccatgggcacagcaggt gtatactacgtgatggtcacatctactctttccatctttcagggaaagataaagccgacctac agatttcggtgcttgaatgtcattttgtggttgggattctgggctgtgcagctgaatgtctgtc tgtcacgaatctaccttgctgctcattttcctcatcaagttgttgctggagtcctgtcaggcatt gctgttgcagaaactttcagccacatccacagcatctataatgccagcctcaagaaatatttt ctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctgtattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **6** | hGPE | tggaggaagcagaaaggggctggcaggtggaaagatgaggaccagctcatcgtctcat gactatgaggttgctctgatccagaggtgccccctgcctggtggcccaccgccaggaaga |
| | promoter (4 nucleotides difference to hGPE promoter in NG011808.1) | ctcccactgtccctggatgcccagagtgggatgtcaactccatcacttatcaactccttatcc atagggggtattcttcctgaggcgtctcagaaaacagggccctccccatatgctgaccacata atagaacccctcccaactcagagaccctggctgctagctgccctggcatgacccagacagt ggcctttgtatatgtttttagactcaccttgactcacctctgaccatagaaactctcatcccag aggtcactgcaatagttactccacaacagaggcttatctgggtagagggaggctccctacc tatggcccagcagccctgacagtgcagatcacatataccccacgccccagcactgcctgcc acgcatgggcttactttacacccacccacagtcaccaacacattacctgctctccaaggtta ggcgtggcaggagaagtttgcttggaccagcagaaaccatgcagtcaaggacaactgga gtcagcatgggctgggtgcgagcccttggtggggtggggaggagactccaggtcatacct cctggaggatgttttaatcatttccagcatggaatgctgtcaacttttgccacagattcattag ctctgagtttctttttctgtccccagctaccccttacatgtcaatatggacttaatgatgggaa attcaggcaagtttttaaacattttattccccctggctcttatcctcaaaaaatgcatgaatttg gaggcagtggctcatgcctgtaatcccaatgctttgctaggttgaggcgggaggatcactt gaagccaggaatttgagaccagcctgggccgcatagtgagaccccgtttctacaaaaata aataaataaataaataatagtgatatgaagcatgattaaatagccctatttttttaaaatg catgagttcgttacctgattcattccctggttcctttcacagtcctccgtgacccaagtgttagg gttttggtctctctactatttgtaggctgatatatagtatacacacacacacacacacacatat acacacacacagtgtatcttgagctttcttttgtatatctacacacatatgtataagaaagctc aagatatagaagcccttttttcaaaaataactgaaagtttcaaactctttaagtctccagttac cattttgctggtattcttatttggaaccatacattcatcatattgttgcacagtaagactataca ttcattattttgcttaaacgtatgagttaaaacacttggccaggcatggtggttcacacctgta atcccagagctttgggaagccaagactggcagtactcttgagctcaggaattcaagacca gcctgggcaacatggaaaaaccccatctctacaaaagatagaaaaattagccaggcatg gtggcgtgtgcctgtggtcccagctactcaggaggctgaggtgggaggatcacattagcc caggaggttgaggctgcagtgagccgtgattatgccactgcactccagcctgggagacag agtgagaccctgtttcaaaaaaaagagagagaaaatttaaaaaagaaaacaacaccaa gggctgtaactttaaggtcattaaatgaattaatcactgcattcaaaaacgattactttctgg ccctaagagacatgaggccaataccaggaaggggggttgatctcccaaaccagaggcag accctagactctaatacagttaaggaaagaccagcaagatgatagtccccaatacaatag aagttactatattttatttgttgtttttcttttgttttgttttgttttgttttgttttgttttagagactgg ggtcttgctcgattgcccaggctgtagtgcagcggtgggacaatagctcactgcagactcc aactcctgggctcaagcaatcctcctgcctcagcctcctgaatagctgggactacaagggt acaccatcacacacacaccaaaacaatttttttaaatttttgtgtagaaacgagggtcttgctttgt tgcccaggctggtctccaactcctggcttcaagggatcctcccacctcagcctcccaaattgc tgggattacaggtgtgagccaccacaaccagccagaactttactaattttaaaattaagaa cttaaaacttgaatagctagagcaccaagatttttctttgtccccaaataagtgcagttgcag gcatagaaaatctgacatctttgcaagaatcatcgtggatgtagactctgtcctgtgtctctg gcctggtttcggggaccaggagggcagacccttgcactgccaagaagcatgccaaagtta atcattggccctgctgagtacatggccgatcaggctgtttttgtgtgcctgtttttctattttacg taaatcaccctgaacatgtttgcatcaacctactggtgatgcacctttgatcaatacattttag acaaacgtggtttttgagtccaaagatcagggctgggttgacctgaatactggatacaggg catataaaacaggggcaaggcacagactc |
| **7** | hAAT promoter + ApoE enhancer | aggctcagaggcacacaggagtttctgggctcaccctgccccttccaacccctcagttccc atcctccagcagctgtttgtgtgctgcctctgaagtccacactgaacaaacttcagcctactc atgtccctaaaatgggcaaacattgcaagcagcaaacagcaaacacacagccctccctgc ctgctgaccttggagctggggcagaggtcagagacctctctgggcccatgccacctccaac atccactcgaccccttggaatttcggtggagaggagcagaggttgtcctggcgtggtttag gtagtgtgagaggggtacccggggatcttgctaccagtggaacagccactaaggattctg cagtgagagcagagggccagctaagtggtactctcccagagactgtctgactcacgccac cccctccaccttggacacaggacgctgtggtttctgagccaggtacaatgactcctttcggt aagtgcagtggaagctgtacactgcccaggcaaagcgtccgggcagcgtaggcgggcg actcagatcccagccagtggacttagcccctgtttgctcctccgataactggggtgaccttg gttaatattcaccagcagcctcccccgttgcccctctggatccactgcttaaatacggacga |
| | | ggacagggccctgtctcctcagcttcaggcaccaccactgacctgggacagtgaat |
| **8** | hAAT promoter | gtacccggggatcttgctaccagtggaacagccactaaggattctgcagtgagagcagag ggccagctaagtggtactctcccagagactgtctgactcacgccacccctccaccttggac acaggacgctgtggtttctgagccaggtacaatgactcctttcggtaagtgcagtggaagc tgtacactgcccaggcaaagcgtccgggcagcgtaggcgggcgactcagatcccagcca gtggacttagcccctgtttgctcctccgataactggggtgaccttggttaatattcaccagca gcctcccccgttgcccctctggatccactgcttaaatacggacgaggacagggccctgtctc ctcagcttcaggcaccaccactgacctgggacagtgaat |
| **9** | ApoE enhancer | aggctcagaggcacacaggagtttctgggctcaccctgccccttccaacccctcagttccc atcctccagcagctgtttgtgtgctgcctctgaagtccacactgaacaaacttcagcctactc atgtccctaaaatgggcaaacattgcaagcagcaaacagcaaacacacagccctccctgc ctgctgaccttggagctggggcagaggtcagagacctctctgggcccatgccacctccaac atccactcgaccccttggaatttcggtggagaggagcagaggttgtcctggcgtggtttag gtagtgtgagaggg |
| **10** | hGPE_HBB2_ hG6PCwt_bG H (expression cassette used in the examples having hGPE promoter) | tggaggaagcagaaaggggctggcaggtggaaagatgaggaccagctcatcgtctcat gactatgaggttgctctgatccagaggtgccccctgcctggtggcccaccgccaggaaga ctcccactgtccctggatgcccagagtgggatgtcaactccatcacttatcaactccttatcc ataggggtattcttcctgaggcgtctcagaaaacagggccctccccatatgctgaccacata atagaacccctcccaactcagagaccctggctgctagctgccctggcatgacccagacagt ggcctttgtatatgtttttagactcaccttgactcacctctgaccatagaaactctcatcccag aggtcactgcaatagttactccacaacagaggcttatctgggtagagggaggctccctacc tatggcccagcagccctgacagtgcagatcacatataccccacgccccagcactgcctgcc acgcatgggcttactttacacccacccacagtcaccaacacattacctgctctccaaggtta ggcgtggcaggagaagtttgcttggaccagcagaaaccatgcagtcaaggacaactgga gtcagcatgggctgggtgcgagcccttggtggggtggggaggagactccaggtcatacct cctggaggatgttttaatcatttccagcatggaatgctgtcaacttttgccacagattcattag ctctgagtttctttttctgtccccagctaccccttacatgtcaatatggacttaatgatgggaa attcaggcaagtttttaaacattttattcccccctggctcttatcctcaaaaaatgcatgaatttg gaggcagtggctcatgcctgtaatcccaatgctttgctaggttgaggcgggaggatcactt gaagccaggaatttgagaccagcctgggccgcatagtgagaccccgtttctacaaaaata aataaataaataataaataatagtgatatgaagcatgattaaatagccctatttttaaaatg catgagttcgttacctgattcattccctggttcctttcacagtcctccgtgacccaagtgttagg gttttggtctctctactatttgtaggctgatatatagtatacacacacacacacacacatat acacacacacagtgtatcttgagctttcttttgtatatctacacacatatgtataagaaagctc aagatatagaagccctttttcaaaaataactgaaagtttcaaactctttaagtctccagttac cattttgctggtattcttatttggaaccatacattcatcatattgttgcacagtaagactataca ttcattattttgcttaaacgtatgagttaaaacacttggccaggcatggtggttcacacctgta atcccagagctttgggaagccaagactggcagtactcttgagctcaggaattcaagacca gcctgggcaacatggaaaaaccccatctctacaaaagatagaaaaattagccaggcatg gtggcgtgtgcctgtggtcccagctactcaggaggctgaggtgggaggatcacattagcc caggaggttgaggctgcagtgagccgtgattatgccactgcactccagcctgggagacag agtgagaccctgtttcaaaaaaaagagagagaaaatttaaaaagaaaacaacaccaa gggctgtaactttaaggtcattaaatgaattaatcactgcattcaaaaacgattactttctgg ccctaagagacatgaggccaataccaggaaggggggttgatctcccaaaccagaggcag accctagactctaatacagttaaggaaagaccagcaagatgatagtccccaatacaatag aagttactatattttatttgttgtttttcttttgttttgttttgttttgttttgttttgttttagagactgg ggtcttgctcgattgcccaggctgtagtgcagcggtgggacaatagctcactgcagactcc aactcctgggctcaagcaatcctcctgcctcagcctcctgaatagctgggactacaaggt |
| | | acaccatcacacacaccaaaacaatttttaaatttttgtgtagaaacgagggtcttgctttgt tgcccaggctggtctccaactcctggcttcaaggatcctcccacctcagcctcccaaattgc tgggattacaggtgtgagccaccacaaccagccagaactttactaattttaaaattaagaa cttaaaacttgaatagctagagcaccaagattttcttgtccccaaataagtgcagttgcag gcatagaaaatctgacatctttgcaagaatcatcgtggatgtagactctgtcctgtgtctctg gcctggtttcggggaccaggagggcagacccttgcactgccaagaagcatgccaaagtta atcattggccctgctgagtacatggccgatcaggctgtttttgtgtgcctgtttttctattttacg taaatcaccctgaacatgtttgcatcaacctactggtgatgcacctttgatcaatacattttag acaaacgtggtttttgagtccaaagatcagggctgggttgacctgaatactggatacaggg catataaaacaggggcaaggcacagactcgggacccttgatgttttctttccccttcttttcta tggttaagttcatgtcataggaaggggagaagtaacagggtacacatattgaccaaatca gggtaatttgcatttgtaattttaaaaaatgctttcttcttttaatatactttttttgtttatcttattt ctaatactttccctaatctctttctttcagggcaatattgatacaatgtatcttgcctctttgcacc attctaaagaataacagtgataatttctgggttaaggcaatagcaatatttctgcatataaat atttctgcatataaattgtaactgatgtaagaggtttcatattgctaatagcagctacaatcca gctaccattctgcttttattttctggttgggataaggctggattattctgagtccaagctaggc ccttttgctaatcttgttcatacctcttatcttcctcccacagctcctgggcaacctgctggtctct ctgctggcccatcactttggcaaagcacgcgtgccaccatggaggaaggaatgaatgttct ccatgactttgggatccagtcaacacattacctccaggtgaattaccaagactcccaggact ggttcatcttggtgtccgtgatcgcagacctcaggaatgccttctacgtcctcttccccatctg gttccatcttcaggaagctgtgggcattaaactcctttgggtagctgtgattggagactggct caacctcgtctttaagtggattctctttggacagcgtccatactggtgggttttggatactgac tactacagcaacacttccgtgcccctgataaagcagttccctgtaacctgtgagactggacc agggagcccctctggccatgccatgggcacagcaggtgtatactacgtgatggtcacatct actctttccatctttcagggaaagataaagccgacctacagatttcggtgcttgaatgtcattt tgtggttgggattctgggctgtgcagctgaatgtctgtctgtcacgaatctaccttgctgctca ttttcctcatcaagttgttgctggagtcctgtcaggcattgctgttgcagaaactttcagccac atccacagcatctataatgccagcctcaagaaatatttctcattaccttcttcctgttcagctt cgccatcggattttatctgctgctcaagggactgggtgtagacctcctgtggactctggaga aagcccagaggtggtgcgagcagccagaatgggtccacattgacaccacaccctttgcca gcctcctcaagaacctgggcacgctctttggcctggggctggctctcaactccagcatgtac agggagagctgcaaggggaaactcagcaagtggctcccattccgcctcagctctattgta gcctccctcgtcctcctgcacgtctttgactccttgaaacccccatcccaagtcgagctggtct tctacgtcttgtccttctgcaagagtgcggtagtgcccctggcatccgtcagtgtcatcccta ctgcctcgcccaggtcctgggccagccgcacaagaagtcgttgtaaagatctgaattcacc gcgggtttaaactgtgccttctagttgccagccatctgttgtttgcccctcccccgtgccttcct tgaccctggaaggtgccactcccactgtcctttcctaataaaatgaggaaattgcatcgcatt gtctgagtaggtgtcattctattctggggggtggggtggggctagc |
| **11** | hAAT_HBB2_ hG6PCwt_HB B2 (expression cassette used in the examples having hAAT promoter) | ggatcaaggctcagaggcacacaggagtttctgggctcaccctgccccttccaacccctc agttcccatcctccagcagctgtttgtgtgctgcctctgaagtccacactgaacaaacttcag cctactcatgtccctaaaatgggcaaacattgcaagcagcaaacagcaaacacacagccc tccctgcctgctgaccttggagctggggcagaggtcagagacctctctgggcccatgccac ctccaacatccactcgaccccttggaatttcggtggagaggagcagaggttgtcctggcgt ggtttaggtagtgtgagaggggtacccggggatcttgctaccagtggaacagccactaag gattctgcagtgagagcagaagggccagctaagtggtactctcccagagactgtctgactca cgccacccccctccaccttggacacaggacgctgtggtttctgagccaggtacaatgactcct ttcggtaagtgcagtggaagctgtacactgcccaggcaaagcgtccgggcagcgtaggc gggcgactcagatcccagccagtggacttagcccctgtttgctcctccgataactggggtg accttggttaatattcaccagcagcctcccccgttgcccctctggatccactgcttaaatacg gacgaggacagggccctgtctcctcagcttcaggcaccaccactgacctgggacagtgaa tagatcctgagaacttcagggtgagtctatgggacccttgatgttttctttccccttcttttctat ggttaagttcatgtcataggaaggggagaagtaacagggtacacatattgaccaaatcag |
| | | ggtaattttgcatttgtaatttaaaaaatgctttcttcttttaatatactttttttgtttatcttatttc taatactttccctaatctctttctttcagggcaatattgatacaatgtatcttgcctctttgcacca ttctaaagaataacagtgataatttctgggttaaggcaatagcaatatttctgcatataaata tttctgcatataaattgtaactgatgtaagaggtttcatattgctaatagcagctacaatccag ctaccattctgcttttattttctggttgggataaggctggattattctgagtccaagctaggccc ttttgctaatcttgttcatacctcttatcttcctcccacagctcctgggcaacctgctggtctctct gctggcccatcactttggcaaagcacgcgtgccaccatggaggaaggaatgaatgttctcc atgactttgggatccagtcaacacattacctccaggtgaattaccaagactcccaggactg gttcatcttggtgtccgtgatcgcagacctcaggaatgccttctacgtcctcttccccatctgg ttccatcttcaggaagctgtgggcattaaactcctttgggtagctgtgattggagactggctc aacctcgtctttaagtggattctctttggacagcgtccatactggtgggttttggatactgact actacagcaacacttccgtgcccctgataaagcagttccctgtaacctgtgagactggacca gggagcccctctggccatgccatgggcacagcaggtgtatactacgtgatggtcacatcta ctctttccatctttcagggaaagataaagccgacctacagatttcggtgcttgaatgtcatttt gtggttgggattctgggctgtgcagctgaatgtctgtctgtcacgaatctaccttgctgctcat tttcctcatcaagttgttgctggagtcctgtcaggcattgctgttgcagaaactttcagccaca tccacagcatctataatgccagcctcaagaaatatttctcattaccttcttcctgttcagcttc gccatcggattttatctgctgctcaagggactgggtgtagacctcctgtggactctggagaa agcccagaggtggtgcgagcagccagaatgggtccacattgacaccacaccctttgccag cctcctcaagaacctgggcacgctctttggcctggggctggctctcaactccagcatgtaca gggagagctgcaaggggaaactcagcaagtggctcccattccgcctcagctctattgtag cctccctcgtcctcctgcacgtctttgactccttgaaacccccatcccaagtcgagctggtctt ctacgtcttgtccttctgcaagagtgcggtagtgcccctggcatccgtcagtgtcatcccta ctgcctcgcccaggtcctgggccagccgcacaagaagtcgttgtaaagatctgaattcacc ccaccagtgcaggctgcctatcagaaagtggtggctggtgtggctaatgccctggcccaca agtatcactaagctcgctttcttgctgtccaatttctattaaaggttcctttgttccctaagtcca actactaaactgggggatattatgaagggccttgagcatctggattctgcctaataaaaaa catttattttcattgcaatgatgtatttaaattatttctgaatattttactaaaaagggaatgtgg gaggtcagtgcatttaaaacataaagaaatgaagagctagttcaaaccttgggaaaatac actatatcttaaactccatgaaagaaggtgaggctgcaaacagctaatgcacattggcaac agcccctgatgcctatgccttattcatccctcagaaaaggattcaagtagaggcttgatttgg aggttaaagtttggctatgctgtattttacattacttattgttttagctgtcctcatgaatgtcttt tcactacccatttgcttatcctgcatctctcagccttgactccactcagttctcttgcttagagat accacctttcccctgaagtgttccttccatgttttacggcgagatggtttctcctcgcctggcca ctcagccttagttgtctctgttgtcttatagaggtctacttgaagaaggaaaaacaggggggc atggtttgactgtcctgtgagcccttcttccctgcctcccccactcacagtgacccggaatccc tcgacatggcag |
| **12** | hG6PCmut (amino acid) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSCIVASLVLLHVFDSLKPPSQVELVFYVLSFCKSAVVPLASVSVIPYCL AQVLGQPHKKSL |
| **13** | Modified G6Pase-a (R3K) | Atggagaaaggaatgaatgttctccatgactttgggatccagtcaacacattacctccaggt gaattaccaagactcccaggactggttcatcttggtgtccgtgatcgcagacctcaggaatg ccttctacgtcctcttccccatctggttccatcttcaggaagctgtgggcattaaactcctttgg gtagctgtgattggagactggctcaacctcgtctttaagtggattctctttggacagcgtcca tactggtgggttttggatactgactactacagcaacacttccgtgcccctgataaagcagttc cctgtaacctgtgagactggaccagggagcccctctggccatgccatgggcacagcaggt gtatactacgtgatggtcacatctactctttccatctttcagggaaagataaagccgacctac agatttcggtgcttgaatgtcattttgtggttgggattctgggctgtgcagctgaatgtctgtc |
| | | tgtcacgaatctaccttgctgctcattttcctcatcaagttgttgctggagtcctgtcaggcatt gctgttgcagaaactttcagccacatccacagcatctataatgccagcctcaagaaatatttt ctcattaccttcttcctgttcagcttcgccatcggattttatctgctgctcaaggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **14** | Modified G6Pase-a (Q54R) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcgtgaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcatttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaaggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacaggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **15** | Modified G6Pase-a (Q139R) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcggggaaagataaagccgaccta cagatttcggtgcttgaatgtcattttgtggttgggattctgggctgtgcagctgaatgtctgt ctgtcacgaatctaccttgctgctcatttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaaggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacaggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **16** | Modified G6Pase-a (I142K) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagaaaaagccgaccta |
| | | cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **17** | Modified G6Pase-a (S196R) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttccgccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **18** | Modified G6Pase-a (H199Q) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccagagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **19** | Modified G6Pase-a (Q242R) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg |
| | | tgtatactacgtgatggtcacatctactctttccatctttcagggaaagataaagccgaccta cagatttcggtgcttgaatgtcattttgtggttggattctggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagccaggaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **20** | Modified G6Pase-a (Q247R) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcggccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **21** | Modified G6Pase-a (L292F) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggttc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **22** | Modified G6Pase-a (S298C) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt |
| | | ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaaggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctgcattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **23** | Modified G6Pase-a (A301V) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcatttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaaggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacaggagagctgcaaggggaaactcagcaagtggctc ccattccgcctcagctctattgtagtgtccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcctgggccagccgcacaagaag tcgttgtaa |
| **24** | Modified G6Pase-a (V318T) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaaactgagctggtcttctacgtcttgtccttctgcaagagtgcggtagtgcccct ggcatccgtcagtgtcatcccctactgcctcgcccaggtcctgggccagccgcacaagaag tcgttgtaa |
| **25** | Modified G6Pase-a (V324T) | atggaggaaggaatgaatgttctccatgactttgggatccagtcaacacattacctccaggt gaattaccaagactcccaggactggttcatcttggtgtccgtgatcgcagacctcaggaatg ccttctacgtcctcttccccatctggttccatcttcaggaagctgtgggcattaaactcctttgg gtagctgtgattggagactggctcaacctcgtctttaagtggattctctttggacagcgtcca |
| | | tactggtgggttttggatactgactactacagcaacacttccgtgcccctgataaagcagttc cctgtaacctgtgagactggaccagggagcccctctggccatgccatgggcacagcaggt gtatactacgtgatggtcacatctactctttccatctttcagggaaagataaagccgacctac agatttcggtgcttgaatgtcattttgtggttgggattctgggctgtgcagctgaatgtctgtc tgtcacgaatctaccttgctgctcattttcctcatcaagttgttgctggagtcctgtcaggcatt gctgttgcagaaactttcagccacatccacagcatctataatgccagcctcaagaaatatttt ctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacaccttgtccttctgcaagagtgcggtagtgcccct ggcatccgtcagtgtcatcccctactgcctcgcccaggtcctgggccagccgcacaagaag tcgttgtaa |
| **26** | Modified G6Pase-a (V332A) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggcagtgcccct ggcatccgtcagtgtcatcccctactgcctcgcccaggtcctgggccagccgcacaagaag tcgttgtaa |
| **27** | Modified G6Pase-a (Q347R) | Atggaggaaggaatgaatgttctccatgactttgggatccagtcaacacattacctccagg tgaattaccaagactcccaggactggttcatcttggtgtccgtgatcgcagacctcaggaat gccttctacgtcctcttccccatctggttccatcttcaggaagctgtgggcattaaactcctttg ggtagctgtgattggagactggctcaacctcgtctttaagtggattctctttggacagcgtcc atactggtgggttttggatactgactactacagcaacacttccgtgcccctgataaagcagtt ccctgtaacctgtgagactggaccagggagcccctctggccatgccatgggcacagcagg tgtatactacgtgatggtcacatctactctttccatctttcagggaaagataaagccgaccta cagatttcggtgcttgaatgtcattttgtggttgggattctgggctgtgcagctgaatgtctgt ctgtcacgaatctaccttgctgctcattttcctcatcaagttgttgctggagtcctgtcaggcat tgctgttgcagaaactttcagccacatccacagcatctataatgccagcctcaagaaatattt tctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccgggtcctgggccagccgcacaagaag tcgttgtaa |
| **28** | Modified G6Pase-a (L349F) | atggaggaaggaatgaatgttctccatgactttgggatccagtcaacacattacctccaggt gaattaccaagactcccaggactggttcatcttggtgtccgtgatcgcagacctcaggaatg ccttctacgtcctcttccccatctggttccatcttcaggaagctgtgggcattaaactcctttgg |
| | | gtagctgtgattggagactggctcaacctcgtctttaagtggattctctttggacagcgtcca tactggtgggttttggatactgactactacagcaacacttccgtgcccctgataaagcagttc cctgtaacctgtgagactggaccagggagcccctctggccatgccatgggcacagcaggt gtatactacgtgatggtcacatctactctttccatctttcagggaaagataaagccgacctac agatttcggtgcttgaatgtcattttgtggttgggattctgggctgtgcagctgaatgtctgtc tgtcacgaatctaccttgctgctcattttcctcatcaagttgttgctggagtcctgtcaggcatt gctgttgcagaaactttcagccacatccacagcatctataatgccagcctcaagaaatatttt ctcattaccttcttcctgttcagcttcgccatcggattttatctgctgctcaagggactgggtgt agacctcctgtggactctggagaaagcccagaggtggtgcgagcagccagaatgggtcc acattgacaccacaccctttgccagcctcctcaagaacctgggcacgctctttggcctgggg ctggctctcaactccagcatgtacagggagagctgcaaggggaaactcagcaagtggctc ccattccgcctcagctctattgtagcctccctcgtcctcctgcacgtctttgactccttgaaacc cccatcccaagtcgagctggtcttctacgtcttgtccttctgcaagagtgcggtagtgcccct ggcatccgtcagtgtcatcccctactgcctcgcccaggtcttcggccagccgcacaagaag tcgttgtaa |
| | | |
| **29** | Modified G6Pase-a (R3K) | MEKGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **30** | Modified G6Pase-a (Q54R) | MEEGMNVLHDFGIQSTHYLQVNYQDSQDWFILVSVIADLRNAFYVLFP IWFHLREAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **31** | Modified G6Pase-a (Q139R) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFRGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **32** | Modified G6Pase-a (I142K) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKKKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **33** | Modified G6Pase-a (S196R) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE |
| | | TFRHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **34** | Modified G6Pase-a (H199Q) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIQSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **35** | Modified G6Pase-a (Q242R) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKARRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **36** | Modified G6Pase-a (Q247R) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CERPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVLGQPHKKSL |
| **37** | Modified G6Pase-a (L292F) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWFP FRLSSIVASLVLLHVFDSLKPPSQVELVFYVLSFCKSAVVPLASVSVIPYCL AQVLGQPHKKSL |
| **38** | Modified G6Pase-a (S298C) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSCIVASLVLLHVFDSLKPPSQVELVFYVLSFCKSAVVPLASVSVIPYCL AQVLGQPHKKSL |
| **39** | Modified G6Pase-a (A301V) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVVSLVLLHVFDSLKPPSQVELVFYVLSFCKSAVVPLASVSVIPYCL AQVLGQPHKKSL |
| **40** | Modified G6Pase-a (V318T) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQTELVFYVLSFCKSAVVPLASVSVIPYCL AQVLGQPHKKSL |
| **41** | Modified G6Pase-a (V324T) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYTLSFCKSAVVPLASVSVIPYCL AQVLGQPHKKSL |
| **42** | Modified G6Pase-a (V332A) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAAVPLASVSVIPYCL AQVLGQPHKKSL |
| **43** | Modified G6Pase-a (Q347R) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL ARVLGQPHKKSL |
| **44** | Modified G6Pase-a (L349F) | MEEGMNVLHDFGIQSTHYLQVNYQDSQDWFILVSVIADLRNAFYVLFP IWFHLQEAVGIKLLWVAVIGDWLNLVFKWILFGQRPYWWVLDTDYYS NTSVPLIKQFPVTCETGPGSPSGHAMGTAGVYYVMVTSTLSIFQGKIKP TYRFRCLNVILWLGFWAVQLNVCLSRIYLAAHFPHQVVAGVLSGIAVAE TFSHIHSIYNASLKKYFLITFFLFSFAIGFYLLLKGLGVDLLWTLEKAQRW CEQPEWVHIDTTPFASLLKNLGTLFGLGLALNSSMYRESCKGKLSKWLP FRLSSIVASLVLLHVFDSLKPPSQVELVFYVLSFCKSAVVPLASVSVIPYCL AQVFGQPHKKSL |

## Claims

1. A nucleic acid construct for the expression of a glucose-6-phosphatase-a (G6Pase-a) in a cell, the construct comprising a nucleic acid sequence encoding the G6Pase-a, wherein the nucleic acid sequence encoding the G6Pase-a is operably linked to a human alpha-1 antitrypsin (hAAT) promoter.

2. The nucleic acid construct according to claim 1, wherein the G6Pase-a has an amino acid sequence at least 90% identical to SEQ ID NO: 1.

3. The nucleic acid construct according to any of claims 1 or 2, wherein the nucleic acid sequence encoding the G6Pase-a comprises a nucleotide sequence having at least 90% identity with SEQ ID NO: 2.

4. The nucleic acid construct according to any of claims 1 to 3, wherein the hAAT promoter comprises a nucleotide sequence having at least 90% identity with SEQ ID NO: 8.

5. The nucleic acid construct according to any of claims 1 to 4, wherein said construct comprises a nucleotide sequence having at least 90% identity with SEQ ID NO: 7 or comprises the nucleotide sequence SEQ ID NO: 11.

6. The nucleic acid construct according to any of claims 1 to 5, wherein the cell is a liver cell, a kidney cell or an intestine cell.

7. A vector comprising the nucleic acid construct of any of claims 1 to 6.

8. The vector of claim 7, which is a viral vector, such as lentiviral vector or adeno-associated virus (AAV) vector.

9. The vector of any of claim 7 or 8, which is an AAV vector.

10. The vector of any of claims 7 to 9, which is an AAV serotype 8 (AAV8) vector.

11. A cell transformed with the nucleic acid molecule of any one of claims 1 to 6 or the vector of any one of claims 7 to 10.

12. The cell according to claim 11, which is a liver cell, an intestinal cell or a kidney cell.

13. A composition comprising the nucleic acid construct of any one of claims 1 to 6, the vector of any one of claims 7 to 10, or the cell of any one of claims 11 to 12.

14. The nucleic acid construct of any one of claims 1 to 6, the vector of any one of claims 7 to 10, the cell according to any one of claims 11 to 12 or the composition according to any one of claim 13, for use as a medicament.

15. The nucleic acid construct of any one of claims 1 to 6, the vector of any one of claims 7 to 10, the cell according to any one of claims 11 to 12 or the composition according to any one of claim 13, for use in the treatment of glycogen storage disease Ia (GSD-Ia).


SEQUENCE LISTING

<110> GENETHON
      INSTITUT NATIONAL DE LA RECHERCHE ET DE LA SANTE MEDICALE
      UNIVERSITE EVRY VAL DESSONNE
      UNIVERSITE CLAUDE BERNARD LYON 1

<120> Vectors encoding a glucose-6-phosphatase (G6Pase-a) for gene therapy

<130> 1H274440_CAS 4

<160> 44

<170> BiSSAP 1.3.6

<210> 1
<211> 357
<212> PRT
<213> Homo sapiens

<220>
<223> hG6PCwt (amino acid)

<400> 1

<210> 2
<211> 1074
<212> DNA
<213> Homo sapiens


<220>
<223> hG6PCwt (nucleic acid) (encoding SEQ ID NO:1)

<400> 2
atggaggaag gaatgaatgt tctccatgac tttgggatcc agtcaacaca ttacctccag      60

gtgaattacc aagactccca ggactggttc atcttggtgt ccgtgatcgc agacctcagg     120

aatgccttct acgtcctctt ccccatctgg ttccatcttc aggaagctgt gggcattaaa     180

ctcctttggg tagctgtgat tggagactgg ctcaacctcg tctttaagtg gattctcttt     240

ggacagcgtc catactggtg ggttttggat actgactact acagcaacac ttccgtgccc     300

ctgataaagc agttccctgt aacctgtgag actggaccag ggagcccctc tggccatgcc     360

atgggcacag caggtgtata ctacgtgatg gtcacatcta ctctttccat ctttcaggga     420

aagataaagc cgacctacag atttcggtgc ttgaatgtca ttttgtggtt gggattctgg     480

gctgtgcagc tgaatgtctg tctgtcacga atctaccttg ctgctcattt tcctcatcaa     540

gttgttgctg gagtcctgtc aggcattgct gttgcagaaa ctttcagcca catccacagc     600

atctataatg ccagcctcaa gaaatatttt ctcattacct tcttcctgtt cagcttcgcc     660

atcggatttt atctgctgct caagggactg ggtgtagacc tcctgtggac tctggagaaa     720

gcccagaggt ggtgcgagca gccagaatgg gtccacattg acaccacacc ctttgccagc     780

ctcctcaaga acctgggcac gctctttggc ctggggctgg ctctcaactc cagcatgtac     840

agggagagct gcaaggggaa actcagcaag tggctcccat tccgcctcag ctctattgta     900

gcctccctcg tcctcctgca cgtctttgac tccttgaaac ccccatccca agtcgagctg     960

gtcttctacg tcttgtcctt ctgcaagagt gcggtagtgc ccctggcatc cgtcagtgtc    1020

atcccctact gcctcgccca ggtcctgggc cagccgcaca agaagtcgtt gtaa          1074


<210> 3
<211> 1074
<212> DNA
<213> Artificial Sequence


<220>

<223> hG6PCco (nucleic acid) (Codon optimized - encoding SEQ ID NO :
      1)(

<400> 3
atggaggagg gaatgaatgt gctgcacgac ttcgggattc agtctaccca ctatctccaa      60

gtcaattatc aagacagtca agattggttc atcctcgtct ccgttatagc tgacctcagg     120

aatgcttttt atgtactgtt cccaatatgg tttcatctgc aagaagcagt tggaattaaa     180

ctgctgtggg tggccgtaat cggagactgg ttgaacctgg tgttcaaatg gatccttttt     240

ggtcagaggc catattggtg ggtcttggac accgactatt atagcaacac atctgtaccc     300

ctgataaagc aattccctgt aacgtgtgaa actgggcctg ggtcacccag tggacacgcg     360

atggggactg ccggggttta ctacgtaatg gttactagca cgcttagcat tttccagggc     420

aagatcaaac cgacttatcg cttccgatgc ctcaatgtga tcctctggct gggattctgg     480

gcggttcaac tgaacgtatg cctctcacgg atttatctgg ctgcacattt cccgcatcaa     540

gtagtggctg gtgtgttgtc tggcatagca gtggctgaaa cattctcaca cattcattct     600

atttacaacg cttcattgaa aaaatacttt ctgattactt tttttctttt cagctttgca     660

attggctttt acttgctgct taaaggtctt ggcgtcgacc tgctctggac tcttgagaaa     720

gcgcaacgct ggtgcgaaca acccgagtgg gtccacatag atacgacccc gttcgcgtct     780

cttctgaaaa atctcggtac cctcttcgga ctgggacttg ctttgaattc ttcaatgtat     840

cgggaatcct gcaagggcaa actgtccaag tggttgccct ttcgcctttc aagcattgtc     900

gcttcccttg tgctcctgca cgtcttcgac tcactgaagc cgccgagcca agtcgagctc     960

gtgttttacg tgttgtcttt ctgtaaaagt gccgtcgtgc cactcgccag tgtgagcgtg    1020

ataccttatt gcctcgcaca ggtgcttggg cagcctcata agaagtctct gtaa          1074

<210> 4
<211> 1074
<212> DNA
<213> Artificial Sequence

<220>
<223> hG6PCmut (nucleic acid) (identical to NM000151.4 SEQ ID NO: 2
      except for a c >g mutation at position 893)

<400> 4
atggaggaag gaatgaatgt tctccatgac tttgggatcc agtcaacaca ttacctccag      60

gtgaattacc aagactccca ggactggttc atcttggtgt ccgtgatcgc agacctcagg     120

aatgccttct acgtcctctt ccccatctgg ttccatcttc aggaagctgt gggcattaaa     180

ctcctttggg tagctgtgat tggagactgg ctcaacctcg tctttaagtg gattctcttt     240

ggacagcgtc catactggtg ggttttggat actgactact acagcaacac ttccgtgccc     300

ctgataaagc agttccctgt aacctgtgag actggaccag ggagcccctc tggccatgcc     360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctgtattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 5
<211> 1074
<212> DNA
<213> Artificial Sequence

<220>
<223> hG6PCmutco (Nucleic acid)(Codon optimized - encoding SEQ ID NO :
      12)

<400> 5
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctgtattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 6
<211> 2700
<212> DNA
<213> Artificial Sequence

<220>
<223> hGPE promoter (4 nucleotides difference to hGPE promoter in
      NG011808.1)

<400> 6
tggaggaagc agaaaggggc tggcaggtgg aaagatgagg accagctcat cgtctcatga 60

ctatgaggtt gctctgatcc agaggtgccc cctgcctggt ggcccaccgc caggaagact 120

cccactgtcc ctggatgccc agagtgggat gtcaactcca tcacttatca actccttatc 180

cataggggta ttcttcctga ggcgtctcag aaaacagggc cctccccata tgctgaccac 240

ataatagaac ccctcccaac tcagagaccc tggctgctag ctgccctggc atgacccaga 300

cagtggcctt tgtatatgtt tttagactca ccttgactca cctctgacca tagaaactct 360

catcccagag gtcactgcaa tagttactcc acaacagagg cttatctggg tagagggagg 420

ctccctacct atggcccagc agccctgaca gtgcagatca catatacccc acgccccagc 480

actgcctgcc acgcatgggc ttactttaca cccacccaca gtcaccaaca cattacctgc 540

tctccaaggt taggcgtggc aggagaagtt tgcttggacc agcagaaacc atgcagtcaa 600

ggacaactgg agtcagcatg ggctgggtgc gagcccttgg tggggtgggg aggagactcc 660

aggtcatacc tcctggagga tgttttaatc atttccagca tggaatgctg tcaacttttg 720

ccacagattc attagctctg agtttctttt ttctgtcccc agctacccct tacatgtcaa 780

tatggactta atgatgggaa attcaggcaa gtttttaaac attttattcc ccctggctct 840

tatcctcaaa aaatgcatga atttggaggc agtggctcat gcctgtaatc ccaatgcttt 900

gctaggttga ggcgggagga tcacttgaag ccaggaattt gagaccagcc tgggccgcat 960

agtgagaccc cgtttctaca aaaataaata aataaataat aaataatagt gatatgaagc 1020

atgattaaat agccctattt tttaaaatgc atgagttcgt tacctgattc attccctggt 1080

tcctttcaca gtcctccgtg acccaagtgt tagggttttg gtctctctac tatttgtagg 1140

ctgatatata gtatacacac acacacacac acacatatac acacacacag tgtatcttga 1200

gctttctttt gtatatctac acacatatgt ataagaaagc tcaagatata gaagcccttt 1260

ttcaaaaata actgaaagtt tcaaactctt taagtctcca gttaccattt tgctggtatt 1320

cttatttgga accatacatt catcatattg ttgcacagta agactataca ttcattattt 1380

tgcttaaacg tatgagttaa aacacttggc caggcatggt ggttcacacc tgtaatccca 1440

gagctttggg aagccaagac tggcagtact cttgagctca ggaattcaag accagcctgg 1500

gcaacatgga aaaaccccat ctctacaaaa gatagaaaaa ttagccaggc atggtggcgt 1560

gtgcctgtgg tcccagctac tcaggaggct gaggtgggag gatcacatta gcccaggagg 1620

ttgaggctgc agtgagccgt gattatgcca ctgcactcca gcctgggaga cagagtgaga 1680

ccctgtttca aaaaaaagag agagaaaatt taaaaaagaa aacaacacca agggctgtaa 1740

ctttaaggtc attaaatgaa ttaatcactg cattcaaaaa cgattacttt ctggccctaa 1800

gagacatgag gccaatacca ggaagggggt tgatctccca aaccagaggc agaccctaga 1860

ctctaataca gttaaggaaa gaccagcaag atgatagtcc ccaatacaat agaagttact 1920

atattttatt tgttgttttt cttttgtttt gttttgtttt gttttgtttt gttttagaga 1980

ctggggtctt gctcgattgc ccaggctgta gtgcagcggt gggacaatag ctcactgcag 2040

actccaactc ctgggctcaa gcaatcctcc tgcctcagcc tcctgaatag ctgggactac 2100

aagggtacac catcacacac accaaaacaa ttttttaaat ttttgtgtag aaacgagggt 2160

cttgctttgt tgcccaggct ggtctccaac tcctggcttc aagggatcct cccacctcag 2220

cctcccaaat tgctgggatt acaggtgtga gccaccacaa ccagccagaa ctttactaat 2280

tttaaaatta agaacttaaa acttgaatag ctagagcacc aagatttttc tttgtcccca 2340

aataagtgca gttgcaggca tagaaaatct gacatctttg caagaatcat cgtggatgta 2400

gactctgtcc tgtgtctctg gcctggtttc ggggaccagg agggcagacc cttgcactgc 2460

caagaagcat gccaaagtta atcattggcc ctgctgagta catggccgat caggctgttt 2520

ttgtgtgcct gtttttctat tttacgtaaa tcaccctgaa catgtttgca tcaacctact 2580

ggtgatgcac ctttgatcaa tacattttag acaaacgtgg tttttgagtc caaagatcag 2640

ggctgggttg acctgaatac tggatacagg gcatataaaa caggggcaag gcacagactc 2700

<210> 7
<211> 727
<212> DNA
<213> Artificial Sequence

<220>
<223> ApoE enhancer + hAAT promoter (composed by ApoE enhancer U32510.1
      and hAAT promoter NG_008290.1)

<400> 7
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg ggtacccggg gatcttgcta ccagtggaac agccactaag 360

gattctgcag tgagagcaga gggccagcta agtggtactc tcccagagac tgtctgactc 420

acgccacccc ctccaccttg gacacaggac gctgtggttt ctgagccagg tacaatgact 480

cctttcggta agtgcagtgg aagctgtaca ctgcccaggc aaagcgtccg ggcagcgtag 540

gcgggcgact cagatcccag ccagtggact tagcccctgt ttgctcctcc gataactggg 600

gtgaccttgg ttaatattca ccagcagcct cccccgttgc ccctctggat ccactgctta 660

aatacggacg aggacagggc cctgtctcct cagcttcagg caccaccact gacctgggac 720

agtgaat 727

<210> 8
<211> 406
<212> DNA
<213> Artificial Sequence

<220>
<223> hAAT promoter

<400> 8
gtacccgggg atcttgctac cagtggaaca gccactaagg attctgcagt gagagcagag 60

ggccagctaa gtggtactct cccagagact gtctgactca cgccaccccc tccaccttgg 120

acacaggacg ctgtggtttc tgagccaggt acaatgactc ctttcggtaa gtgcagtgga 180

agctgtacac tgcccaggca aagcgtccgg gcagcgtagg cgggcgactc agatcccagc 240

cagtggactt agcccctgtt tgctcctccg ataactgggg tgaccttggt taatattcac 300

cagcagcctc ccccgttgcc cctctggatc cactgcttaa atacggacga ggacagggcc 360

ctgtctcctc agcttcaggc accaccactg acctgggaca gtgaat 406

<210> 9
<211> 321
<212> DNA
<213> Artificial Sequence

<220>
<223> ApoE enhancer

<400> 9
aggctcagag gcacacagga gtttctgggc tcaccctgcc cccttccaac ccctcagttc 60

ccatcctcca gcagctgttt gtgtgctgcc tctgaagtcc acactgaaca aacttcagcc 120

tactcatgtc cctaaaatgg gcaaacattg caagcagcaa acagcaaaca cacagccctc 180

cctgcctgct gaccttggag ctggggcaga ggtcagagac ctctctgggc ccatgccacc 240

tccaacatcc actcgacccc ttggaatttc ggtggagagg agcagaggtt gtcctggcgt 300

ggtttaggta gtgtgagagg g 321

<210> 10
<211> 4492
<212> DNA
<213> Artificial Sequence

<220>
<223> hGPE_HBB2_hG6PCwt_bGH (expression cassette used in the examples
      having hGPE promoter)

<400> 10
tggaggaagc agaaaggggc tggcaggtgg aaagatgagg accagctcat cgtctcatga 60

ctatgaggtt gctctgatcc agaggtgccc cctgcctggt ggcccaccgc caggaagact 120

cccactgtcc ctggatgccc agagtgggat gtcaactcca tcacttatca actccttatc 180

cataggggta ttcttcctga ggcgtctcag aaaacagggc cctccccata tgctgaccac 240

ataatagaac ccctcccaac tcagagaccc tggctgctag ctgccctggc atgacccaga 300

cagtggcctt tgtatatgtt tttagactca ccttgactca cctctgacca tagaaactct 360

catcccagag gtcactgcaa tagttactcc acaacagagg cttatctggg tagagggagg 420

ctccctacct atggcccagc agccctgaca gtgcagatca catatacccc acgccccagc 480

actgcctgcc acgcatgggc ttactttaca cccacccaca gtcaccaaca cattacctgc 540

tctccaaggt taggcgtggc aggagaagtt tgcttggacc agcagaaacc atgcagtcaa 600

ggacaactgg agtcagcatg ggctgggtgc gagcccttgg tggggtgggg aggagactcc 660

aggtcatacc tcctggagga tgttttaatc atttccagca tggaatgctg tcaacttttg 720

ccacagattc attagctctg agtttctttt ttctgtcccc agctacccct tacatgtcaa 780

tatggactta atgatgggaa attcaggcaa gtttttaaac attttattcc ccctggctct 840

tatcctcaaa aaatgcatga atttggaggc agtggctcat gcctgtaatc ccaatgcttt 900

gctaggttga ggcgggagga tcacttgaag ccaggaattt gagaccagcc tgggccgcat 960

agtgagaccc cgtttctaca aaaataaata aataaataat aaataatagt gatatgaagc 1020

atgattaaat agccctattt tttaaaatgc atgagttcgt tacctgattc attccctggt 1080

tcctttcaca gtcctccgtg acccaagtgt tagggttttg gtctctctac tatttgtagg 1140

ctgatatata gtatacacac acacacacac acacatatac acacacacag tgtatcttga 1200

gctttctttt gtatatctac acacatatgt ataagaaagc tcaagatata gaagcccttt 1260

ttcaaaaata actgaaagtt tcaaactctt taagtctcca gttaccattt tgctggtatt 1320

cttatttgga accatacatt catcatattg ttgcacagta agactataca ttcattattt 1380

tgcttaaacg tatgagttaa aacacttggc caggcatggt ggttcacacc tgtaatccca 1440

gagctttggg aagccaagac tggcagtact cttgagctca ggaattcaag accagcctgg 1500

gcaacatgga aaaaccccat ctctacaaaa gatagaaaaa ttagccaggc atggtggcgt 1560

gtgcctgtgg tcccagctac tcaggaggct gaggtgggag gatcacatta gcccaggagg 1620

ttgaggctgc agtgagccgt gattatgcca ctgcactcca gcctgggaga cagagtgaga 1680

ccctgtttca aaaaaaagag agagaaaatt taaaaaagaa aacaacacca agggctgtaa 1740

ctttaaggtc attaaatgaa ttaatcactg cattcaaaaa cgattacttt ctggccctaa 1800

gagacatgag gccaatacca ggaagggggt tgatctccca aaccagaggc agaccctaga 1860

ctctaataca gttaaggaaa gaccagcaag atgatagtcc ccaatacaat agaagttact 1920

atattttatt tgttgttttt cttttgtttt gttttgtttt gttttgtttt gttttagaga 1980

ctggggtctt gctcgattgc ccaggctgta gtgcagcggt gggacaatag ctcactgcag 2040

actccaactc ctgggctcaa gcaatcctcc tgcctcagcc tcctgaatag ctgggactac 2100

aagggtacac catcacacac accaaaacaa ttttttaaat ttttgtgtag aaacgagggt 2160

cttgctttgt tgcccaggct ggtctccaac tcctggcttc aagggatcct cccacctcag 2220

cctcccaaat tgctgggatt acaggtgtga gccaccacaa ccagccagaa ctttactaat 2280

tttaaaatta agaacttaaa acttgaatag ctagagcacc aagatttttc tttgtcccca 2340

aataagtgca gttgcaggca tagaaaatct gacatctttg caagaatcat cgtggatgta 2400

gactctgtcc tgtgtctctg gcctggtttc ggggaccagg agggcagacc cttgcactgc 2460

caagaagcat gccaaagtta atcattggcc ctgctgagta catggccgat caggctgttt 2520

ttgtgtgcct gtttttctat tttacgtaaa tcaccctgaa catgtttgca tcaacctact 2580

ggtgatgcac ctttgatcaa tacattttag acaaacgtgg tttttgagtc caaagatcag 2640

ggctgggttg acctgaatac tggatacagg gcatataaaa caggggcaag gcacagactc 2700

gggacccttg atgttttctt tccccttctt ttctatggtt aagttcatgt cataggaagg 2760

ggagaagtaa cagggtacac atattgacca aatcagggta attttgcatt tgtaatttta 2820

aaaaatgctt tcttctttta atatactttt ttgtttatct tatttctaat actttcccta 2880

atctctttct ttcagggcaa tattgataca atgtatcttg cctctttgca ccattctaaa 2940

gaataacagt gataatttct gggttaaggc aatagcaata tttctgcata taaatatttc 3000

tgcatataaa ttgtaactga tgtaagaggt ttcatattgc taatagcagc tacaatccag 3060

ctaccattct gcttttattt tctggttggg ataaggctgg attattctga gtccaagcta 3120

ggcccttttg ctaatcttgt tcatacctct tatcttcctc ccacagctcc tgggcaacct 3180

gctggtctct ctgctggccc atcactttgg caaagcacgc gtgccaccat ggaggaagga 3240

atgaatgttc tccatgactt tgggatccag tcaacacatt acctccaggt gaattaccaa 3300

gactcccagg actggttcat cttggtgtcc gtgatcgcag acctcaggaa tgccttctac 3360

gtcctcttcc ccatctggtt ccatcttcag gaagctgtgg gcattaaact cctttgggta 3420

gctgtgattg gagactggct caacctcgtc tttaagtgga ttctctttgg acagcgtcca 3480

tactggtggg ttttggatac tgactactac agcaacactt ccgtgcccct gataaagcag 3540

ttccctgtaa cctgtgagac tggaccaggg agcccctctg gccatgccat gggcacagca 3600

ggtgtatact acgtgatggt cacatctact ctttccatct ttcagggaaa gataaagccg 3660

acctacagat ttcggtgctt gaatgtcatt ttgtggttgg gattctgggc tgtgcagctg 3720

aatgtctgtc tgtcacgaat ctaccttgct gctcattttc ctcatcaagt tgttgctgga 3780

gtcctgtcag gcattgctgt tgcagaaact ttcagccaca tccacagcat ctataatgcc 3840

agcctcaaga aatattttct cattaccttc ttcctgttca gcttcgccat cggattttat 3900

ctgctgctca agggactggg tgtagacctc ctgtggactc tggagaaagc ccagaggtgg 3960

tgcgagcagc cagaatgggt ccacattgac accacaccct ttgccagcct cctcaagaac 4020

ctgggcacgc tctttggcct ggggctggct ctcaactcca gcatgtacag ggagagctgc 4080

aaggggaaac tcagcaagtg gctcccattc cgcctcagct ctattgtagc ctccctcgtc 4140

ctcctgcacg tctttgactc cttgaaaccc ccatcccaag tcgagctggt cttctacgtc 4200

ttgtccttct gcaagagtgc ggtagtgccc ctggcatccg tcagtgtcat cccctactgc 4260

ctcgcccagg tcctgggcca gccgcacaag aagtcgttgt aaagatctga attcaccgcg 4320

ggtttaaact gtgccttcta gttgccagcc atctgttgtt tgcccctccc ccgtgccttc 4380

cttgaccctg gaaggtgcca ctcccactgt cctttcctaa taaaatgagg aaattgcatc 4440

gcattgtctg agtaggtgtc attctattct ggggggtggg gtgggggcta gc 4492

<210> 11
<211> 3153
<212> DNA
<213> Artificial Sequence

<220>
<223> hAAT_HBB2_hG6PCwt_HBB2 (expression cassette used in the examples
      having hAAT promoter)

<400> 11
ggatcaaggc tcagaggcac acaggagttt ctgggctcac cctgccccct tccaacccct 60

cagttcccat cctccagcag ctgtttgtgt gctgcctctg aagtccacac tgaacaaact 120

tcagcctact catgtcccta aaatgggcaa acattgcaag cagcaaacag caaacacaca 180

gccctccctg cctgctgacc ttggagctgg ggcagaggtc agagacctct ctgggcccat 240

gccacctcca acatccactc gaccccttgg aatttcggtg gagaggagca gaggttgtcc 300

tggcgtggtt taggtagtgt gagaggggta cccggggatc ttgctaccag tggaacagcc 360

actaaggatt ctgcagtgag agcagagggc cagctaagtg gtactctccc agagactgtc 420

tgactcacgc caccccctcc accttggaca caggacgctg tggtttctga gccaggtaca 480

atgactcctt tcggtaagtg cagtggaagc tgtacactgc ccaggcaaag cgtccgggca 540

gcgtaggcgg gcgactcaga tcccagccag tggacttagc ccctgtttgc tcctccgata 600

actggggtga ccttggttaa tattcaccag cagcctcccc cgttgcccct ctggatccac 660

tgcttaaata cggacgagga cagggccctg tctcctcagc ttcaggcacc accactgacc 720

tgggacagtg aatagatcct gagaacttca gggtgagtct atgggaccct tgatgttttc 780

tttccccttc ttttctatgg ttaagttcat gtcataggaa ggggagaagt aacagggtac 840

acatattgac caaatcaggg taattttgca tttgtaattt taaaaaatgc tttcttcttt 900

taatatactt ttttgtttat cttatttcta atactttccc taatctcttt ctttcagggc 960

aatattgata caatgtatct tgcctctttg caccattcta aagaataaca gtgataattt 1020

ctgggttaag gcaatagcaa tatttctgca tataaatatt tctgcatata aattgtaact 1080

gatgtaagag gtttcatatt gctaatagca gctacaatcc agctaccatt ctgcttttat 1140

tttctggttg ggataaggct ggattattct gagtccaagc taggcccttt tgctaatctt 1200

gttcatacct cttatcttcc tcccacagct cctgggcaac ctgctggtct ctctgctggc 1260

ccatcacttt ggcaaagcac gcgtgccacc atggaggaag gaatgaatgt tctccatgac 1320

tttgggatcc agtcaacaca ttacctccag gtgaattacc aagactccca ggactggttc 1380

atcttggtgt ccgtgatcgc agacctcagg aatgccttct acgtcctctt ccccatctgg 1440

ttccatcttc aggaagctgt gggcattaaa ctcctttggg tagctgtgat tggagactgg 1500

ctcaacctcg tctttaagtg gattctcttt ggacagcgtc catactggtg ggttttggat 1560

actgactact acagcaacac ttccgtgccc ctgataaagc agttccctgt aacctgtgag 1620

actggaccag ggagcccctc tggccatgcc atgggcacag caggtgtata ctacgtgatg 1680

gtcacatcta ctctttccat ctttcaggga aagataaagc cgacctacag atttcggtgc 1740

ttgaatgtca ttttgtggtt gggattctgg gctgtgcagc tgaatgtctg tctgtcacga 1800

atctaccttg ctgctcattt tcctcatcaa gttgttgctg gagtcctgtc aggcattgct 1860

gttgcagaaa ctttcagcca catccacagc atctataatg ccagcctcaa gaaatatttt 1920

ctcattacct tcttcctgtt cagcttcgcc atcggatttt atctgctgct caagggactg 1980

ggtgtagacc tcctgtggac tctggagaaa gcccagaggt ggtgcgagca gccagaatgg 2040

gtccacattg acaccacacc ctttgccagc ctcctcaaga acctgggcac gctctttggc 2100

ctggggctgg ctctcaactc cagcatgtac agggagagct gcaaggggaa actcagcaag 2160

tggctcccat tccgcctcag ctctattgta gcctccctcg tcctcctgca cgtctttgac 2220

tccttgaaac ccccatccca agtcgagctg gtcttctacg tcttgtcctt ctgcaagagt 2280

gcggtagtgc ccctggcatc cgtcagtgtc atcccctact gcctcgccca ggtcctgggc 2340

cagccgcaca agaagtcgtt gtaaagatct gaattcaccc caccagtgca ggctgcctat 2400

cagaaagtgg tggctggtgt ggctaatgcc ctggcccaca agtatcacta agctcgcttt 2460

cttgctgtcc aatttctatt aaaggttcct ttgttcccta agtccaacta ctaaactggg 2520

ggatattatg aagggccttg agcatctgga ttctgcctaa taaaaaacat ttattttcat 2580

tgcaatgatg tatttaaatt atttctgaat attttactaa aaagggaatg tgggaggtca 2640

gtgcatttaa aacataaaga aatgaagagc tagttcaaac cttgggaaaa tacactatat 2700

cttaaactcc atgaaagaag gtgaggctgc aaacagctaa tgcacattgg caacagcccc 2760

tgatgcctat gccttattca tccctcagaa aaggattcaa gtagaggctt gatttggagg 2820

ttaaagtttg gctatgctgt attttacatt acttattgtt ttagctgtcc tcatgaatgt 2880

cttttcacta cccatttgct tatcctgcat ctctcagcct tgactccact cagttctctt 2940

gcttagagat accacctttc ccctgaagtg ttccttccat gttttacggc gagatggttt 3000

ctcctcgcct ggccactcag ccttagttgt ctctgttgtc ttatagaggt ctacttgaag 3060

aaggaaaaac agggggcatg gtttgactgt cctgtgagcc cttcttccct gcctccccca 3120

ctcacagtga cccggaatcc ctcgacatgg cag 3153

<210> 12
<211> 357
<212> PRT
<213> Artificial Sequence

<220>
<223> hG6PCmut (amino acid)

<400> 12
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr

115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Cys Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 13
<211> 1074
<212> DNA
<213> Artificial Sequence

<220>
<223> Modified G6Pase-a (R3K)

<400> 13
atggagaaag gaatgaatgt tctccatgac tttgggatcc agtcaacaca ttacctccag      60

gtgaattacc aagactccca ggactggttc atcttggtgt ccgtgatcgc agacctcagg     120

aatgccttct acgtcctctt ccccatctgg ttccatcttc aggaagctgt gggcattaaa     180

ctcctttggg tagctgtgat tggagactgg ctcaacctcg tctttaagtg gattctcttt     240

ggacagcgtc catactggtg ggttttggat actgactact acagcaacac ttccgtgccc     300

ctgataaagc agttccctgt aacctgtgag actggaccag ggagcccctc tggccatgcc     360

atgggcacag caggtgtata ctacgtgatg gtcacatcta ctctttccat ctttcaggga     420

aagataaagc cgacctacag atttcggtgc ttgaatgtca ttttgtggtt gggattctgg     480

gctgtgcagc tgaatgtctg tctgtcacga atctaccttg ctgctcattt tcctcatcaa     540

gttgttgctg gagtcctgtc aggcattgct gttgcagaaa ctttcagcca catccacagc     600

atctataatg ccagcctcaa gaaatatttt ctcattacct tcttcctgtt cagcttcgcc     660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 14
<211> 1074
<212> DNA
<213> Artificial Sequence

<220>
<223> Modified G6Pase-a (Q54R)

<400> 14
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc gtgaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 15
<211> 1074
<212> DNA
<213> Artificial Sequence

<220>
<223> Modified G6Pase-a (Q139R)

<400> 15
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcgggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 16
<211> 1074
<212> DNA
<213> Artificial Sequence

<220>
<223> Modified G6Pase-a (I142K)

<400> 16
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagaaaaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 17
<211> 1074
<212> DNA
<213> Artificial Sequence

<220>
<223> Modified G6Pase-a (S196R)

<400> 17
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttccgcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 18
<211> 1074
<212> DNA
<213> Artificial Sequence

<220>
<223> Modified G6Pase-a (H199Q)

<400> 18
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccagagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 19

<211> 1074
<212> DNA
<213> Artificial Sequence

<220>
<223> Modified G6Pase-a (Q242R)

<400> 19
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gccaggaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 20
<211> 1074
<212> DNA
<213> Artificial Sequence

<220>
<223> Modified G6Pase-a (Q247R)

<400> 20
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagcg gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 21
<211> 1074
<212> DNA
<213> Artificial Sequence

<220>
<223> Modified G6Pase-a (L292F)

<400> 21
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggttcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 22
<211> 1074
<212> DNA
<213> Artificial Sequence

<220>
<223> Modified G6Pase-a (S298C)

<400> 22
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctgcattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 23
<211> 1074

<212> DNA
<213> Artificial Sequence

<220>
<223> Modified G6Pase-a (A301V)

<400> 23
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gtgtccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 24
<211> 1074
<212> DNA
<213> Artificial Sequence

<220>
<223> Modified G6Pase-a (V318T)

<400> 24
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca aactgagctg 960

gtcttctacg tcttgtcctt ctgcaagagt gcggtagtgc ccctggcatc cgtcagtgtc 1020

atcccctact gcctcgccca ggtcctgggc cagccgcaca agaagtcgtt gtaa 1074

<210> 25
<211> 1074
<212> DNA
<213> Artificial Sequence

<220>
<223> Modified G6Pase-a (V324T)

<400> 25
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctaca ccttgtcctt ctgcaagagt gcggtagtgc ccctggcatc cgtcagtgtc 1020

atcccctact gcctcgccca ggtcctgggc cagccgcaca agaagtcgtt gtaa 1074

<210> 26
<211> 1074
<212> DNA
<213> Artificial Sequence

<220>
<223> Modified G6Pase-a (V332A)

<400> 26
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggcagtgc ccctggcatc cgtcagtgtc 1020

atcccctact gcctcgccca ggtcctgggc cagccgcaca agaagtcgtt gtaa 1074

<210> 27
<211> 1074
<212> DNA

<213> Artificial Sequence

<220>
<223> Modified G6Pase-a (Q347R)

<400> 27
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac ccccatccca agtcgagctg 960

gtcttctacg tcttgtcctt ctgcaagagt gcggtagtgc ccctggcatc cgtcagtgtc 1020

atcccctact gcctcgcccg ggtcctgggc cagccgcaca agaagtcgtt gtaa 1074

<210> 28
<211> 1074
<212> DNA
<213> Artificial Sequence

<220>
<223> Modified G6Pase-a (L349F)

<400> 28
atggaggaag gaatgaatgt tctccatgac tttgggatcc agtcaacaca ttacctccag 60

gtgaattacc aagactccca ggactggttc atcttggtgt ccgtgatcgc agacctcagg 120

aatgccttct acgtcctctt ccccatctgg ttccatcttc aggaagctgt gggcattaaa 180

ctcctttggg tagctgtgat tggagactgg ctcaacctcg tctttaagtg gattctcttt 240

ggacagcgtc catactggtg ggttttggat actgactact acagcaacac ttccgtgccc 300

ctgataaagc agttccctgt aacctgtgag actggaccag ggagcccctc tggccatgcc 360

atgggcacag caggtgtata ctacgtgatg gtcacatcta ctctttccat ctttcaggga 420

aagataaagc cgacctacag atttcggtgc ttgaatgtca ttttgtggtt gggattctgg 480

gctgtgcagc tgaatgtctg tctgtcacga atctaccttg ctgctcattt tcctcatcaa 540

gttgttgctg gagtcctgtc aggcattgct gttgcagaaa ctttcagcca catccacagc 600

atctataatg ccagcctcaa gaaatatttt ctcattacct tcttcctgtt cagcttcgcc 660

atcggatttt atctgctgct caagggactg ggtgtagacc tcctgtggac tctggagaaa 720

gcccagaggt ggtgcgagca gccagaatgg gtccacattg acaccacacc ctttgccagc 780

ctcctcaaga acctgggcac gctctttggc ctggggctgg ctctcaactc cagcatgtac 840

agggagagct gcaaggggaa actcagcaag tggctcccat tccgcctcag ctctattgta 900

gcctccctcg tcctcctgca cgtctttgac tccttgaaac cccatcccca agtcgagctg 960

gtcttctacg tcttgtcctt ctgcaagagt gcggtagtgc ccctggcatc cgtcagtgtc 1020

atcccctact gcctcgccca ggtcttcggc cagccgcaca agaagtcgtt gtaa 1074

<210> 29
<211> 357
<212> PRT
<213> Artificial Sequence

<220>
<223> Modified G6Pase-a (R3K)

<400> 29
Met Glu Lys Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys

195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 30
<211> 357
<212> PRT
<213> Artificial Sequence

<220>
<223> Modified G6Pase-a (Q54R)

<400> 30
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Arg Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr

245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 31
<211> 357
<212> PRT
<213> Artificial Sequence

<220>
<223> Modified G6Pase-a (Q139R)

<400> 31
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Arg Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val

290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 32
<211> 357
<212> PRT
<213> Artificial Sequence

<220>
<223> Modified G6Pase-a (I142K)

<400> 32
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Lys Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro

340                 345                 350
His Lys Lys Ser Leu
        355

<210> 33
<211> 357
<212> PRT
<213> Artificial Sequence

<220>
<223> Modified G6Pase-a (S196R)

<400> 33
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Arg His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 34
<211> 357

<212> PRT
<213> Artificial Sequence

<220>
<223> Modified G6Pase-a (H199Q)

<400> 34
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile Gln Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 35
<211> 357
<212> PRT
<213> Artificial Sequence

<220>
<223> Modified G6Pase-a (Q242R)

<400> 35
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Arg Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 36
<211> 357
<212> PRT
<213> Artificial Sequence

<220>
<223> Modified G6Pase-a (Q247R)

<400> 36
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30

Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Arg Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 37
<211> 357
<212> PRT
<213> Artificial Sequence

<220>
<223> Modified G6Pase-a (L292F)

<400> 37
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80

Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Phe Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 38
<211> 357
<212> PRT
<213> Artificial Sequence

<220>
<223> Modified G6Pase-a (S298C)

<400> 38
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125

Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Cys Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 39
<211> 357
<212> PRT
<213> Artificial Sequence

<220>
<223> Modified G6Pase-a (A301V)

<400> 39
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175

Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Val Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 40
<211> 357
<212> PRT
<213> Artificial Sequence

<220>
<223> Modified G6Pase-a (V318T)

<400> 40
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220

Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Thr Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 41
<211> 357
<212> PRT
<213> Artificial Sequence

<220>
<223> Modified G6Pase-a (V324T)

<400> 41
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270

Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Thr Leu Ser Phe Cys Lys Ser Ala Val Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 42
<211> 357
<212> PRT
<213> Artificial Sequence

<220>
<223> Modified G6Pase-a (V332A)

<400> 42
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320

Val Phe Tyr Val Leu Ser Phe Cys Lys Ser Ala Ala Val Pro Leu Ala
                325                 330                 335
Ser Val Ser Val Ile Pro Tyr Cys Leu Ala Gln Val Leu Gly Gln Pro
            340                 345                 350
His Lys Lys Ser Leu
        355

<210> 43
<211> 357
<212> PRT
<213> Artificial Sequence

<220>
<223> Modified G6Pase-a (Q347R)

<400> 43
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Arg Val Leu Gly Gln Pro
            340                 345                 350
His Lys Lys Ser Leu
        355

<210> 44
<211> 357
<212> PRT
<213> Artificial Sequence

<220>
<223> Modified G6Pase-a (L349F)

<400> 44
Met Glu Glu Gly Met Asn Val Leu His Asp Phe Gly Ile Gln Ser Thr
1               5                   10                  15
His Tyr Leu Gln Val Asn Tyr Gln Asp Ser Gln Asp Trp Phe Ile Leu
            20                  25                  30
Val Ser Val Ile Ala Asp Leu Arg Asn Ala Phe Tyr Val Leu Phe Pro
        35                  40                  45
Ile Trp Phe His Leu Gln Glu Ala Val Gly Ile Lys Leu Leu Trp Val
    50                  55                  60
Ala Val Ile Gly Asp Trp Leu Asn Leu Val Phe Lys Trp Ile Leu Phe
65                  70                  75                  80
Gly Gln Arg Pro Tyr Trp Trp Val Leu Asp Thr Asp Tyr Tyr Ser Asn
                85                  90                  95
Thr Ser Val Pro Leu Ile Lys Gln Phe Pro Val Thr Cys Glu Thr Gly
            100                 105                 110
Pro Gly Ser Pro Ser Gly His Ala Met Gly Thr Ala Gly Val Tyr Tyr
        115                 120                 125
Val Met Val Thr Ser Thr Leu Ser Ile Phe Gln Gly Lys Ile Lys Pro
    130                 135                 140
Thr Tyr Arg Phe Arg Cys Leu Asn Val Ile Leu Trp Leu Gly Phe Trp
145                 150                 155                 160
Ala Val Gln Leu Asn Val Cys Leu Ser Arg Ile Tyr Leu Ala Ala His
                165                 170                 175
Phe Pro His Gln Val Val Ala Gly Val Leu Ser Gly Ile Ala Val Ala
            180                 185                 190
Glu Thr Phe Ser His Ile His Ser Ile Tyr Asn Ala Ser Leu Lys Lys
        195                 200                 205
Tyr Phe Leu Ile Thr Phe Phe Leu Phe Ser Phe Ala Ile Gly Phe Tyr
    210                 215                 220
Leu Leu Leu Lys Gly Leu Gly Val Asp Leu Leu Trp Thr Leu Glu Lys
225                 230                 235                 240
Ala Gln Arg Trp Cys Glu Gln Pro Glu Trp Val His Ile Asp Thr Thr
                245                 250                 255
Pro Phe Ala Ser Leu Leu Lys Asn Leu Gly Thr Leu Phe Gly Leu Gly
            260                 265                 270
Leu Ala Leu Asn Ser Ser Met Tyr Arg Glu Ser Cys Lys Gly Lys Leu
        275                 280                 285
Ser Lys Trp Leu Pro Phe Arg Leu Ser Ser Ile Val Ala Ser Leu Val
    290                 295                 300
Leu Leu His Val Phe Asp Ser Leu Lys Pro Pro Ser Gln Val Glu Leu
305                 310                 315                 320
Val Phe Tyr Val Leu Ser Phe Cys Lys Ser Ala Val Val Pro Leu Ala
                325                 330                 335
Ser Val Ser Val Ile Pro Tyr Cys Leu Ala Gln Val Phe Gly Gln Pro
            340                 345                 350
His Lys Lys Ser Leu
        355